# EUROPÄISCHE PATENTSCHRIFT

(11) **EP 2 600 882 B1**
(45) Veröffentlichungstag und Bekanntmachung des Hinweises auf die Patenterteilung: **09.01.2019**
(21) Anmeldenummer: 11740668.6
(22) Anmeldetag: 05.08.2011
(51) Int. Cl.: A61K 38/00, A61K 39/395, C07K 16/28

(54) **ANTIKÖRPER GEGEN 6-SULFO LacNAc POSITIVE HUMANE DENDRITISCHE ZELLEN UND DEREN VERWENDUNG**
ANTIBODIES AGAINST 6-SULFO-LacNAc-POSITIVE HUMAN DENDRITIC CELLS, AND THEIR USE
ANTICORPS DIRIGÉS CONTRE DES CELLULES DENDRITIQUES HUMAINES 6-SULFO LacNAc-POSITIVES ET UTILISATION DE CEUX-CI

(30) Priorität: 06.08.2010 DE 102010039019
(43) Veröffentlichungstag der Anmeldung: 12.06.2013
(73) Patentinhaber: Technische Universität Dresden, 01062 Dresden (DE)
(72) Erfinder: BACHMANN, Michael, 65779 Kelkheim (DE); GRÄFE, Daniel, 01307 Dresden (DE)
(74) Vertreter: Kailuweit & Uhlemann Patentanwälte Partnerschaft mbB
(86) Internationale Anmeldenummer: PCT/EP2011/063545
(87) Internationale Veröffentlichungsnummer: WO 2012/017085

(56) Entgegenhaltungen:
- WO-A2-99/58678
- SCHWARZER A ED - SCHWARZER ET AL: "Herstellung und Charakterisierung rekombinanter Antikörper für eine adjuvante Immuntherapie PSCA-positiver Tumore", DISSERTATIONSSCHRIFT ZUR ERLANGUNG EINES DOCTOR MEDICINAE (DR.MED.) DER MEDIZINISCHEN FAKULTÄT CARL GUSTAV CARUS DER TECHNISCHEN UNIVERSITÄT DRESDEN, , 1. Januar 2006 (2006-01-01), Seiten 22-24,96, XP002662580, Gefunden im Internet: URL:http://swbplus.bsz-bw.de/bsz276658973i nh.pdf
- SCHAEKEL KNUT ET AL: "6-Sulfo LacNAc, a novel carbohydrate modification of PSGL-1, defines an inflammatory type of human dendritic cells", IMMUNITY, Bd. 17, Nr. 3, September 2002 (2002-09), Seiten 289-301, XP002665315, ISSN: 1074-7613
- SCHMITZ MARC ET AL: "Tumoricidal potential of native blood dendritic cells: Direct tumor cell killing and activation of NK cell-mediated cytotoxicity", JOURNAL OF IMMUNOLOGY, AMERICAN ASSOCIATION OF IMMUNOLOGISTS, US, Bd. 174, Nr. 7, 1. April 2005 (2005-04-01) , Seiten 4127-4134, XP002629625, ISSN: 0022-1767
- SCHAEKEL K ET AL: "A NOVEL DENDRITIC CELL POPULATION IN HUMAN BLOOD: ONE-STEP IMMUNOMAGNETIC ISOLATION BY A SPECIFIC MAB (M-DC8) AND IN VITRO PRIMING OF CYTOTOXIC T LYMPHOCYTES", EUROPEAN JOURNAL OF IMMUNOLOGY, WILEY - V C H VERLAG GMBH & CO. KGAA, DE, Bd. 28, Nr. 12, 1. Dezember 1998 (1998-12-01), Seiten 4084-4093, XP000857207, ISSN: 0014-2980, DOI: 10.1002/(SICI)1521-4141(199812)28:12<4084: :AID-IMMU4084>3.0.CO;2-4
- MENDE INES ET AL: "Highly efficient antigen targeting to M-DC8(+) dendritic cells via Fc gamma RIII/CD16-specific antibody conjugates", INTERNATIONAL IMMUNOLOGY, Bd. 17, Nr. 5, Mai 2005 (2005-05), Seiten 539-547, XP002665314, ISSN: 0953-8178
- SCHAEKEL KNUT ET AL: "Human 6-sulfo LacNAc-expressing dendritic cells are principal producers of early interleukin-12 and are controlled by erythrocytes", IMMUNITY, Bd. 24, Nr. 6, Juni 2006 (2006-06), Seiten 767-777, XP002665318, ISSN: 1074-7613
- CLAUDIA C. BIPPES ET AL: "A Novel Modular Antigen Delivery System for Immuno Targeting of Human 6-sulfo LacNAc-Positive Blood Dendritic Cells (SlanDCs)", PLOS ONE, Bd. 6, Nr. 1, 1. Januar 2011 (2011-01-01), Seite E16315, XP55008843, ISSN: 1932-6203, DOI: 10.1371/journal.pone.0016315
- DE BAEY ANNEGRET ET AL: "Phenotype and function of human dendritic cells derived from M-DC8+ monocytes", EUROPEAN JOURNAL OF IMMUNOLOGY, Bd. 31, Nr. 6, Juni 2001 (2001-06), Seiten 1646-1655, XP002665316, ISSN: 0014-2980
- BIRKHOLZ KATRIN ET AL: "Targeting of DEC-205 on human dendritic cells results in efficient MHC class II-restricted antigen presentation.", BLOOD 30 SEP 2010 LNKD- PUBMED:20566893, Bd. 116, Nr. 13, 21. Juni 2010 (2010-06-21), Seiten 2277-2285, XP002665317, ISSN: 1528-0020
- T. H. MOGENSEN: "Pathogen Recognition and Inflammatory Signaling in Innate Immune Defenses", CLINICAL MICROBIOLOGY REVIEWS, vol. 22, no. 2, 1 April 2009 (2009-04-01), pages 240-273, XP055106343, ISSN: 0893-8512, DOI: 10.1128/CMR.00046-08

## Beschreibung

Die Erfindung betrifft einen Antikörper gegen humane dendritische Zellen, deren Oberfläche den P Selectin Glycoprotein Ligand 1 (PSGL-1) mit der O-gebundenen Glykanstruktur 6-sulfo N-Acetly-Lactosamin (6-sulfo LacNAc) 6-sulfo LacNAc aufweist (hierin auch als "slan-DC" bezeichnet) aufweist und welche die Immunantwort über slan-DC beeinflussen kann. Ferner betrifft die Erfindung eine Zusammensetzung, die diesen Antikörper enthält sowie deren diagnostische und therapeutische Verwendung. Die erfindungsgemäße Zusammensetzung eignet sich zur Anwendung im Bereich der Medizin, der Pharmazie und in der biomedizinischen Forschung.

Fehlregulationen des Immunsystems können zu schwerwiegenden Erkrankungen führen, die sich entweder durch Immunschwäche oder durch überschießende Immunreaktionen gegen eigentlich harmlose oder gar körpereigene Antigene auszeichnen. Gravierende Infektionen oder Tumorerkrankungen stehen in direktem Zusammenhang mit einer inadäquaten Immunantwort.

Der Mechanismus der Entstehung dieser Erkrankungen ist nach wie vor nicht vollständig aufgeklärt, was die Entwicklung kausaler Therapien gegen die genannten Erkrankungen erschwert. Es ist bekannt, dass dendritische Zellen (DC) in der Lage sind, in Abhängigkeit von den Signalen, die sie erhalten, das Immunsystem entweder zu aktivieren oder zu inaktivieren. Die Inaktivierung des Immunsystems wird auch als ein Zustand der Toleranz bezeichnet, die gegenüber einem diskreten Antigen vorliegt.

DC sind in der Lage, das Immunsystem antigenspezifisch zu aktivieren, also eine Immunreaktion gegen ein bestimmtes Antigen zu vermitteln, indem das Antigen durch aktivierte DC präsentiert wird. Wird ein bestimmtes Antigen durch unaktivierte DC präsentiert, so wird eine Immunreaktion gegen das Antigen verhindert und sogar Toleranz gegen das Antigen induziert (Janeway, Immunologie (7. Auflage 2009); Kapitel 7.26 Seite 385).

Dadurch ist es grundsätzlich möglich, durch gezielte Beeinflussung der durch die DC vermittelten Signale, die vorgenannten Erkrankungen therapeutisch zu behandeln. Problematisch ist hierbei, dass DC keine einheitliche Zellpopulation sind und sich verschiedene Subpopulationen der DC unterschiedlich gut eignen, um durch therapeutisch verabreichte Substanzen, die die Signale der DC beeinflussen, eine Linderung des Krankheitsbilds zu bewirken. Dazu bedarf es Systemen, die spezifisch und mit hoher Affinität an dendritische Zellen, nicht jedoch an andere Zellen binden.

WO 2009/061966 offenbart einen Antikörper, der auch als DEC-205 bezeichnet wird, welcher spezifisch humane DC und Epithelzellen bindet.

EP 1 078 060 B1 offenbart Antikörper, die gegen DC gerichtet sind. Einer der offenbarten Antikörper ist der monoklonale IgM Antikörper "M-DC8", der spezifisch an eine definierte Subpopulation der DCs bindet. Aufgrund der spezifischen Bindung des Antikörper M-DC8 an diese Subpopulation der DC, wurden diese zunächst als "M-DC8 DC" bezeichnet (Schäkel et. al., European J. Immunol., 1998, 28, 4084-93). Es wurde später festgestellt, dass sich diese Subpopulation dadurch auszeichnet, dass auf der Oberfläche der DC der P Selectin Glycoprotein Ligand 1 (PSGL-1) die O-gebundene Glykanstruktur 6-sulfo N-Acetly-Lactosamin (6-sulfo LacNAc, "slan") aufweist (Schäkel et al., Immunity, 2002, 17, 289-301; Schmitz M et al. J of Immunol., 2005, 174, 4127 - 4134; Wehner et al., Int J Cancer. 2009, 15, 358-66). Daher wurde diese Subpopulation der DC ab diesem Zeitpunkt als "slan-DC" bezeichnet (Schäkel et al., Immunity, 2006, 24, 767-77). Es wurde festgestellt, dass diese Subpopulation zur Beeinflussung des Immunsystems besonders geeignet ist (s. a. Mende al. Int. Immunol., 2005, 17 (5): 539-547).

Es ist ein weiterer monoklonaler IgM Antikörper bekannt, der spezifisch an slan-DC bindet und auch als DD2 bezeichnet wird (Schäkel et al., Immunity, 2002, 17, 289-301).

Die Antikörper M-DC8 und DD2 weisen folgende Sequenzen auf:

**Tabelle 1:**

| | Aminosäuresequenz der variablen Region der schweren Kette | Aminosäuresequenz der variablen Region der leichten Kette |
|---|---|---|
| DD2 | SEQ ID No. 19 | SEQ ID No. 20 |
| M-DC8 | SEQ ID No. 21 | SEQ ID No. 22 |

Problematisch für eine therapeutische Anwendung von IgM Antikörpern in monoklonaler Form ist die Handhabbarkeit. IgM Antikörper lassen sich wegen ihres hohen Molekulargewichtes nicht sicher von kleinen Viruspartikeln abtrennen. Ihre schlechte Löslichkeit erschwert ihre Aufreinigung, Lagerung und Applikation als Arzneimittel. Es können nur IgM Antikörper in der Form von Antikörperfragmenten, insbesondere Einzelkettenantikörpern eingesetzt werden, die eine ausreichende Bindungsaffinität eines einzelnen Monomeren aufweisen.

Die EP 1 078 060 B1 offenbart ferner die therapeutische Verwendung der Antikörper, die gegen DC gerichtet sind, zum Targeting von Zielzellen. Dabei wird durch bispezifische Antikörper, die jeweils gegen ein Oberflächenantigen auf DC und gegen ein Oberflächenantigen auf Zielzellen gerichtet sind, eine Rekrutierung der Zielzellen zu den DC ermöglicht. Durch diese Anwendung sollen insbesondere Tumorzellen an DC gebunden werden, um die Aufnahme der Tumorzelle zu verursachen. Dadurch werden antigene Epitope der Tumorzellen bzw. allgemein der Zielzellen auf der Oberfläche der DC präsentiert und sollen eine spezifische Immunantwort gegen die Zielzellen verursachen. Kritisch ist hierbei, dass zur Auslösung dieser Immunantwort gegen die Zielzellen ein weiterer Stimulus notwendig ist, der zur Aktivierung der DC führt. Mit bisherigen Methoden, bei denen ein Stimulus (hierin auch "Co-Stimulus", da dieser immer in Kombination mit dem Antikörper angewendet wird) separat verabreicht wird, welcher z.B. an Pattern Recognition Rezeptoren (PRR) auf DC bindet und dadurch die DC aktiviert, kann kein gezielter Transport dieses Stimulus an die DC erzeugt werden.

Dadurch muss, um einen Effekt zu erzielen, eine große Menge dieses Stimulus systemisch verabreicht werden. Diese systemische Anwendung hat den Nachteil, dass dadurch nicht ausschließlich DC beeinflusst werden, sondern auch andere Zellen des Immunsystems durch die Stimuli beeinflusst und aktiviert werden können, was zu unerwünschten Nebenreaktionen führt. Wird hingegen zu wenig Stimulus verabreicht, werden DC nicht ausreichend aktiviert und können die gewünschte Wirkung nicht vermitteln, sondern bewirken gar das Gegenteil, eine Induktion peripherer Toleranz induzieren.

Um über DC Toleranz zu vermitteln, müssen die DC im inaktivierten Zustand vorliegen, eine gleichzeitige Verabreichung eines Co-Stimulus wird nicht vorgenommen. Aufgrund der Vielzahl an möglichen Antigenen ist es dabei von hoher Bedeutung, Therapiesysteme bereitzustellen, die den gezielten Transport verschiedener Antigene an DC mit einem geringen Herstellungsaufwand zu ermöglichen.

In der Dissertationsschrift von Adrian Schwarzer 2006 (Medizinische Fakultät Carl Gustav Carus der Technischen Universität Dresden) wird ein bispezifischer Antikörper 4B6 x 7F5 beschrieben, wobei eine Funktionalität gegen PSCA gerichtet ist ("7F5") und die andere gegen ein Peptid ("4B6").

Aufgabe der Erfindung ist es, Antikörper bereitzustellen, die für die Anwendung in einem Therapiesystem geeignet sind, dass dazu geeignet ist, Co-Stimuli, die DC aktivieren, gleichzeitig mit dem Antigen gezielt an slan-DC zu transportieren und die bei geringem Herstellungsaufwand den gezielten Transport zahlreicher verschiedener Antigene an slan-DC ermöglichen.

Die Aufgabe wird erfindungsgemäß gelöst durch einen Antikörper gemäß Anspruch 1.

Für therapeutische Anwendungen ist es wünschenswert, wenn die Antikörper möglichst effektiv das Antigen an die slan-DC transportieren. Besonders vorteilhaft eignen sich Antikörper mit einer hohen Bindungsaffinität zur Bindung an slan-DC. Die erfindungsgemäßen anti-slan Antikörper weisen komplementaritätsbestimmende Regionen (complementarity determining regions, CDRs) auf, die die folgenden Aminosäuresequenzen umfassen:
a) variable Region der schweren Kette (V_{H}):
   CDR1 TYGVH (SEQ ID No. 1),
   CDR2 VIWSGGGTDFNVAFXS (SEQ ID No. 2),
      wobei X ausgewählt ist aus M, L, F, oder I, vorzugsweise aus M oder I,
   CDR3 RTTNDGNYAFAY (SEQ ID No. 5) und
b) variable Region der leichten Kette (V_{L}):
   CDR1 RSSQNILHSDGXTYLE (SEQ ID No. 6),
      wobei X ausgewählt ist aus S, T, N, Q, H, K oder R, vorzugsweise aus S oder N,
   CDR2 KVSNRFS (SEQ ID No. 9) und
   CDR3 FQGSHVPWT (SEQ ID No. 10).

Die vorgenannten CDR-Regionen sind in den erfindungsgemäßen Antikörpern in dieser Kombination enthalten. Die erfindungsgemäßen Antikörper liegen bevorzugt als monoklonale Antikörper, als Antikörperfragmente oder in der Form von Fusionsproteinen, insbesondere als bispezifische Antikörper, vor. Bevorzugt sind monovalente Antikörper in der Form von scFv-Fragmenten, F(ab')₂. Weiter bevorzugt sind bispezifische Antikörper in der Form von single chain bispecific Diabodys (scBsDb) oder single chain bispecific Tandem Antikörpern (scBsTaFv). Vorteilhaft binden die erfindungsgemäßen anti-slan Antikörper in der Form von scFv-Fragmenten mit einer deutlich höheren Affinität an slan-DC als Antikörper, die aus dem Stand der Technik bekannt sind.

Die erfindungsgemäßen Antikörper mit obengenannten den CDR-Regionen gemäß SEQ ID No. 1, 2, 5, 6, 9 und 10 binden spezifisch an humane dendritische Zellen binden, deren Oberfläche den P Selectin Glycoprotein Ligand 1 (PSGL-1) mit der O-gebundenen Glykanstruktur 6-sulfo N-Acetly-Lactosamin (6-sulfo LacNAc) aufweist. Erfindungsgemäße Antikörper sind somit anti-slan Antikörper.

Es sind erfindungsgemäße Antikörper bevorzugt, in denen die CDR2 der variablen Region der schweren Kette die Aminosäuresequenz gemäß SEQ ID No. 3 umfasst und CDR1 der variablen Region der leichten Kette die Aminosäuresequenz gemäß SEQ ID No. 7 umfasst (diese Antikörper werden hierin auch als "MB4" bezeichnet). Vorzugsweise umfasst dabei die variable Region der schweren Kette die Aminosäuresequenz gemäß SEQ ID No. 12 (entsprechende Nukleinsäuresequenz gemäß SEQ ID No. 11) und die variable Region der leichten Kette die Aminosäuresequenz gemäß SEQ ID No. 14 (entsprechende Nukleinsäuresequenz gemäß SEQ ID No. 13), gegebenenfalls jeweils als humanisierte Struktur.

Es sind ferner Antikörper bevorzugt, in denen die CDR2 der variablen Region der schweren Kette die Aminosäuresequenz gemäß SEQ ID No. 4 umfasst und CDR1 der variablen Region der leichten Kette die Aminosäuresequenz gemäß SEQ ID No. 8 umfasst (diese Antikörper werden hierin auch als "MB6" bezeichnet). Vorzugsweise umfasst dabei die variable Region der schweren Kette die Aminosäuresequenz gemäß SEQ ID No. 16 (entsprechende Nukleinsäuresequenz gemäß SEQ ID No. 15) und die variable Region der leichten Kette die Aminosäuresequenz gemäß SEQ ID No. 18 (entsprechende Nukleinsäuresequenz gemäß SEQ ID No. 17), gegebenenfalls jeweils als humanisierte Struktur.

Die Antikörper wurden durch die Immunisierung von Mäusen mit isolierten humanen slan-DC und anschließender Hybridomafusion erhalten (vgl. Ausführungsbeispiel 1).

Der "anti-slan Antikörper" ist im erfindungsgemäßen Sinn ein Antikörper gemäß Anspruch 1, der spezifisch an die Oberfläche von slan-DC bindet. Der Begriff "Antikörper" im erfindungsgemäßen Sinn umfasst alle Antikörper, Antikörperfragmente und Derivate derselben, die in der Lage sind, spezifisch an ein Antigen zu binden. Als Antikörper zählen die kompletten monoklonalen Antikörper und auch die epitopbindenden Fragmente dieser Antikörper. Hierbei umfassen die epitopbindenden Fragmente (hier auch als Antikörperfragmente oder Antikörperderivate bezeichnet) alle Teile des Antikörpers, die in der Lage sind, an das Antigen zu binden. Antikörperfragmente im Sinne der Erfindung beinhalten, sind aber ausdrücklich nicht limitiert auf, Fab, Fab', F(ab')₂, Fd, Einzelketten (single-chain) variable Fragmente (scFv), Einzelketten-Antikörper, disulfidverlinkte variable Fragmente (sdFv) und Fragmente die entweder eine variable Region der leichten Kette (V_{L}) oder eine variable Region der schweren Kette (V_{H}) beinhalten. Weiterhin zählen dazu rekombinant hergestellte Antikörper, wie Diabodies, Triabodies und Tetrabodies. Antikörperfragmente enthalten die variablen Regionen entweder allein oder in Kombination weiteren Regionen, die ausgewählt sind aus der Hinge-Region, und dem ersten, zweiten und dritten Bereich der konstanten Region (C_{H}1, C_{H}2, C_{H}3). Ebenfalls durch den Begriff "Antikörper" umfasst sind chimäre Antikörper, bei denen unterschiedliche Teile des Antikörpers aus verschiedenen Spezies stammen, wie beispielsweise Antikörper mit einer murinen variablen Region, welche mit einer humanen konstanten Region kombiniert ist.

Ebenfalls durch den Begriff "Antikörper" umfasst sind humanisierte Antikörper. Das Ziel der Humanisierung von Antikörpern liegt in der Reduktion der Immunogenität eines xenogenen Antikörpers, wie beispielsweise muriner Antikörper, zur Verwendung im humanen System, wobei die volle Bindungsaffinität und die Antigenspezifität erhalten bleibt. Humanisierte Antikörper können auf verschiedenen Wegen hergestellt werden, wie beispielsweise durch Resurfacing und CDR-Grafting. Beim Resurfacing werden durch eine Kombination aus Molecular Modelling, statistischen Analysen und Mutagenese alle nicht-CDR-Regionen auf der Oberfläche des Antikörpers verändert, so dass diese der Oberfläche von Antikörpern des Zielorganismus ähneln. Beim CDR-Grafting werden die CDR-Regionen des zu humanisierenden Antikörpers in humane variable Regionen eingebracht.

Antikörperfragmente sind gegebenenfalls untereinander über einen Linker verknüpft. Der Linker umfasst eine kurze (bevorzugt 10 bis 50 Aminosäurereste lange), flexible Peptidsequenz, die so ausgewählt wird, dass das Antikörperfragment eine derartige dreidimensionale Faltung der V_{L} und V_{H} besitzt, dass es die Antigenspezifität des kompletten Antikörpers aufweist.

Die Bezeichnung "variable Region" ist erfindungsgemäß definiert als die Teile der schweren und leichten Ketten der Antikörper, die sich in ihrer Sequenz zwischen Antikörpern unterscheiden und die Spezifität des Antikörpers und die Bindung an sein Antigen bestimmen. Die Variabilität ist hierbei nicht gleichmäßig in der variablen Region verteilt. Sie ist üblicherweise innerhalb dreier definierter Segmente der variablen Region konzentriert, die als komplementaritätsbestimmende Regionen (CDRs) oder auch hypervariable Regionen bezeichnet werden und in den variablen Regionen sowohl der leichten als auch der schweren Ketten vorhanden sind. Die stärker konservierten Teile der variablen Regionen werden Gerüstregionen (framework regions) genannt. Die variablen Regionen der schweren und leichten Ketten enthalten vier Gerüstregionen, die überwiegend eine beta-Faltblatt Struktur annehmen, wobei jede Gerüstregion mit drei CDRs verbunden ist, die Schleifen bilden, die die beta-Faltblatt-Strukturen verbinden und in manchen Fällen Teil der beta-Faltblatt-Struktur sind. Die CDRs der jeweiligen Kette sind durch die Gerüstregionen in unmittelbare Nähe gebracht und tragen gemeinsam mit den CDRs der anderen Kette zur Bildung der Antigenbindungsregion der Antikörper bei. Bevorzugt umfassen die anti-slan Antikörper mindestens eine variable Region der schweren Kette (V_{H}) und eine variable Region der leichten Kette (V_{L}) in Form eines scFv.

Unter einer spezifischen Bindung eines Antikörpers an ein bestimmtes Antigen im erfindungsgemäßen Sinn wird verstanden, dass ein Antikörper mit einer hohen Affinität an das bestimmte Antigen bindet und mit einer deutlich geringeren Affinität und vorzugsweise nicht an andere Antigene bindet.

Erfindungsgemäße anti-slan Antikörper umfassen die in Tabelle 2 dargestellten, gegebenenfalls humanisierten, Strukturen:

**Tabelle 2:**

| | Aminosäuresequenz der variablen Region der schweren Kette | Aminosäuresequenz der variablen Region der leichten Kette |
|---|---|---|
| MB4 | SEQ ID No. 12 | SEQ ID No. 14 |
| MB6 | SEQ ID No. 16 | SEQ ID No. 18 |

Gegenstand der Erfindung ist auch eine Zusammensetzung, die die folgenden Bestandteile umfasst:
a) mindestens einen erfindungsgemäßen Antikörper, welcher spezifisch an humane dendritische Zellen bindet, deren Oberfläche den P Selectin Glycoprotein Ligand 1 (PSGL-1) mit der O-gebundenen Glykanstruktur 6-sulfo N-Acetly-Lactosamin (6-sulfo LacNAc) aufweist (hierin werden diese Antikörper auch als "anti-slan Antikörper" bezeichnet),
b) mindestens eine an Bindungseinheit, die an einen Co-Stimulus, der an einen für den Co-Stimulus spezifischen Rezeptor auf dendritischen Zellen bindet und dadurch die Modulierung, vorzugsweise die Aktivierung, von besagten dendritischen Zellen bewirkt, und
c) mindestens ein Antigen.

Die Erfindung beruht auf der Idee, dass Co-Stimuli gezielt an dendritische Zellen (DC) transportiert werden können, in dem sie an eine Transportstruktur gebunden sind, die einen anti-slan Antikörper und eine Bindungseinheit enthalten, die den Co-Stimulus direkt bindet. Co-Stimuli bzw. Stimuli sind dazu geeignet, die Modulierung, insbesondere die Aktivierung von dendritischen Zellen zu bewirken. Co-Stimulie, die die Aktivierung von dendritischen Zellen bewirken, werden hierein auch als "aktivierende Co-Stimuli" bezeichnet. Dazu bindet der Co-Stimulus (beispielsweise aktivierende TLR-Liganden, wie Nukleinsäuren) an einen für den Co-Stimulus spezifischen Rezeptor der dendritischen Zellen (beispielsweise Toll like Rezeptoren) und durch die Bindung wird eine Signalkette ausgelöst, die die weitere Entwicklung der DC moduliert. Inhibierende Co-Stimuli bewirken dabei, dass die DC nicht aktiviert wird. Aktivierende Co-Stimuli bewirken dabei, dass die DC aktiviert wird. Der Co-Stimulus ist also ein Ligand, der an einen speziellen Rezeptor der DC bindet. Daher werden hierin die Begriffe "Stimulus", "Co-Stimulus" und "Ligand, der die Modulierung von dendritischen Zellen bewirkt" synonym verwendet. Ferner werden die Begriffe "aktivierender Stimulus", "aktivierender Co-Stimulus" und "Ligand, der die Aktivierung von dendritischen Zellen bewirkt" synonym verwendet. Das Antigen, gegen welches die Immunantwort beeinflusst werden soll, wird ebenfalls gleichzeitig mit der Transportstruktur transportiert. Wenn die dendritische Zelle nach Antigentransport aktiviert wird, wird eine Immunreaktion ausgelöst, die gegen das Antigen gerichtet ist. Wenn die dendritische Zelle nach Antigentransport nicht aktiviert wird, wird Toleranz gegen das Antigen vermittelt und es wird keine pathogene Immunantwort gegen das Antigen induziert.

Die erfindungsgemäße Zusammensetzung umfasst dazu mindestens einen erfindungsgemäßen anti-slan Antikörper, der zur Bindung an humane slan-DC dient, mindestens eine, vorzugsweise mehrere, insbesondere 1 bis 5, bevorzugt 3 bis 5, Bindungseinheiten, die den Co-Stimulus (vorzugsweise einen aktivierenden Co-Stimulus) binden (diese werden hierin auch als "eine an einen Liganden bindende Bindungseinheit" oder auch vereinfacht nur als "Bindungseinheit" bezeichnet) und das zu transportierende Antigen. Die drei Mindestbestandteile sind dabei miteinander verbunden, so dass sie vorzugsweise einen Komplex bilden und nicht als separate Einzelbestandteile vorliegen. Nur durch diese stabile Verbindung ist sichergestellt, dass alle Bestandteile gleichzeitig zu den slan-DC transportiert werden. Die Verbindung der drei Bestandteile kann dabei verschiedenartig ausgestaltet sein, vorzugsweise liegen diese in einem Fusionsprotein vor oder in mehreren Fusionsproteinen, die durch spezifische oder kovalente Bindungen miteinander verbunden sind.

Die Bindungseinheit, die den Co-Stimulus bindet, ist so ausgestaltet, dass sie spezifisch an den Co-Stimulus bindet. Unter einer "Bindungseinheit" im Sinne der Erfindung wird jegliche molekulare Struktur bezeichnet, die definierte Substanzen spezifisch bindet. Je nach Auswahl des zu bindenden Co-Stimulus weist die Bindungseinheit dabei verschiedene Strukturen auf. Ist der Co-Stimulus eine Nukleinsäure, so ist die Bindungseinheit so ausgestaltet, dass sie zur Bindung von Nukleinsäuren geeignet ist. Ist der Co-Stimulus eine bakterielle Glykanstruktur, so ist die Bindungseinheit so ausgestaltet, dass sie zur Bindung von bakteriellen Glykanstrukturen geeignet ist.

Vorzugsweise ist die Bindungseinheit mit dem anti-slan Antikörper und/oder mit dem Antigen über eine kovalente Bindung verbunden. Die kovalente Bindung ist bevorzugt eine Peptidbindung.

Vorzugsweise enthält eine erfindungsgemäße Zusammensetzung eine Bindungseinheit, die einen aktivierenden Co-Stimulus bindet. Co-Stimuli, die zur Aktivierung von DC geeignet sind, sind aus dem Stand der Technik bekannt und umfassen insbesondere Liganden, die an sogenannte Pattern-Recognition Receptors (PRRs) binden. Vorzugsweise ist der Co-Stimulus ein Ligand von C-Typ Lektin-Rezeptoren oder besonders bevorzugt Toll like Rezeptoren. Es ist dabei grundsätzlich unerheblich, ob die Rezeptoren, nach deren Bindung die aktivierenden Signale ausgelöst werden, auf der Zelloberfläche der DC oder intrazellulär lokalisiert sind. Nachdem die Transportstruktur über den anti-slan Antikörper an die slan-DC gebunden hat, bindet der Co-Stimulus entweder vor oder nach der Internalisierung der Transportstruktur an seinen jeweiligen Rezeptor.

Bevorzugt enthält eine erfindungsgemäße Zusammensetzung eine Bindungseinheit, die zur Bindung von Nukleinsäuren geeignet ist. Damit können Co-Stimuli in Form von Nukleinsäuren transportiert werden. Insbesondere können damit TLR-Liganden transportiert werden, bevorzugt ssRNA als Ligand von TLR7 oder TLR8 oder unmethylierte bakterielle DNA (CpG-DNA) als Ligand von TLR9.

Vorzugsweise ist die Bindungseinheit ein Peptid. Die Aminosäuresequenz umfasst bevorzugt 10 - 50 Aminosäuren. Bevorzugt weist das Peptid die Struktur einer alpha-Helix auf. Vorzugsweise umfasst dabei das Linkerpeptid eine Aminosäuresequenz gemäß SEQ ID No. 23, insbesondere eine Aminosäuresequenz gemäß SEQ ID No. 24, oder eine Aminosäuresequenz mit einer Aminosäuresequenzidentität von mindestens 90 %, vorzugsweise mindestens 95 % zu den vorgenannten Sequenzen. Peptide dieser Struktur sind zur Bindung von Nukleinsäuren geeignet.

Bevorzugt ist in erfindungsgemäßen Zusammensetzungen als Bindungseinheit ein Peptid mit einer alpha-Helix-Struktur enthalten, welches dazu geeignet ist, an Nukleinsäuren zu binden.

Das Antigen, welches in einer erfindungsgemäßen Zusammensetzung enthalten ist, ist bevorzugt ein Peptid oder Protein. Die Antigene sind dabei in kompletter Form oder fragmentiert in der erfindungsgemäßen Zusammensetzung enthalten. Bevorzugte Antigene sind Tumorantigene, bakterielle Antigene, Autoantigene oder Allergene. Besonders bevorzugte Antigene sind Tumorantigene, also Antigene, die von Krebszellen produziert werden und die insbesondere auf der Oberfläche von Tumorzellen vorliegen. Davon bevorzugt sind Tumorantigene ausgewählt aus HERs/neu, EGFR, VEGF, CAMPATH 1-Antigen, CD22, CD33, CEA, Prostate Stem Cell Antigen (PSCA), CA-125, HLA-DR, Mucin-1, Survivin, Alpha-1-Fetoprotein, Tyrosinase. Weiter bevorzugte Tumorantigene sind Antigene, die nicht selektiv von Krebszellen produziert werden, jedoch in Tumoren in hohen Konzentrationen vorliegen. Davon besonders bevorzugt sind humane Kernantigene, insbesondere humanes La Protein.

Ferner bevorzugte Antigene sind Allergene, also an sich für den menschlichen Körper harmlose Substanzen, die eine Überempfindlichkeitsreaktion des Immunsystems auslösen. Es sind grundsätzlich alle Allergene in erfindungsgemäßen Zusammensetzungen geeignet, die sich zur Kombination mit dem anti-slan Antikörper und der Bindungseinheit eignen. Besonders bevorzugt werden als Allergene Proteine oder Peptide.

Vorzugsweise liegen der anti-slan Antikörper und die Bindungseinheit als Fusionsprotein vor oder es liegt das Antigen und die Bindungseinheit als Fusionsprotein vor.

Zur Herstellung von rekombinanten Fusionsproteinen (hierin auch vereinfacht als Fusionsproteine bezeichnet) wird ein Fusionsgen erzeugt, indem eine Gensequenz nach Entfernung des Stop-Codons mit einer weiteren Gensequenz fusioniert wird. Bei der Translation dieses Fusionsgens wird ein Fusionsprotein erzeugt. Fusionsproteine umfassen eine Proteindomäne, welche mit einer weiteren Domäne fusioniert ist. Dabei ist mindestens eine Peptiddomäne (als Linkerpeptid) enthalten, ggf. sind weitere Proteindomänen enthalten. Die Funktionalität der einzelnen Proteindomänen bleibt in Fusionsproteinen erhalten, da die funktionellen Domänen von Proteinen meist modular aufgebaut sind, so dass die Aminosäuresequenz einer Proteindomäne, welcher eine bestimmte Funktion zugeordnet ist, vom Rest des Proteins abgetrennt werden kann, ohne dass dessen Funktionalität beeinträchtigt wird.

Besonders bevorzugt liegen anti-slan Antikörper, Bindungseinheit und das Antigen in der erfindungsgemäßen Zusammensetzung als Fusionsprotein vor. Vorzugsweise ist dabei die Bindungseinheit ein vorzugsweise alpha-helikales Peptid, welches über Peptidbindungen mit dem anti-slan Antikörper und dem Antigen verknüpft ist. Davon bevorzugt erfüllt die Bindungseinheit gleichzeitig die Funktion eines Linkerpeptids. Vorzugsweise umfasst dabei das Linkerpeptid eine Aminosäuresequenz gemäß SEQ ID No. 23, insbesondere eine Aminosäuresequenz gemäß SEQ ID No. 24, oder eine Aminosäuresequenz mit einer Aminosäuresequenzidentität von mindestens 90 %, vorzugsweise mindestens 95 % zu den vorgenannten Sequenzen. Um in diesem Fall ein effektives Targeting dieser Fusionsproteine an slan-DC zu bewirken, ist eine besonders hohe Bindungsaffinität an slan-DC erforderlich. Daher eigenen sich in dieser Ausgestaltung besonders ggf. humanisierte anti-slan Antikörper, die die CDR-Regionen der erfindungsgemäßen Antikörper MB4 oder MB6 umfassen.

Erfindungsgemäße Zusammensetzung enthalten vorzugsweise neben den anti-slan Antikörpern keine weiteren Substanzen, die immunphänotypische Oberflächenmerkmale von Zielzellen spezifisch binden. Insbesondere enthalten erfindungsgemäße Fusionsproteine keine Antikörper, die spezifisch an Oberflächenmerkmale, insbesondere Protein- und Glykanstrukturen, von anderen Zellen des Immunsystems, außer DC, binden und auch keine Liganden, die spezifisch an Oberflächenmerkmale, insbesondere Rezeptoren, von Zellen des Immunsystems, außer DC, binden. Die Bestandteile der Zusammensetzung werden somit selektiv an slan-DC transportiert. Erfindungsgemäße Zusammensetzungen haben nicht die Funktion, andere Effektorzellen, wie insbesondere T-Zellen, direkt an DC zu rekrutieren. Daher sind insbesondere keine Antikörper oder Liganden in erfindungsgemäßen Zusammensetzungen enthalten, die an Oberflächenmerkmale von T-Zellen binden.

Vorzugsweise enthält die erfindungsgemäße Zusammensetzung außer den oben genannten Bestandteilen a), b) und c) mindestens eine Bindungseinheit (hierin "weitere Bindungseinheit"), die spezifisch mindestens eines der Bestandteile a), b) oder c) der erfindungsgemäßen Zusammensetzung bindet. Für den Fall, dass die weitere Bindungseinheit an den anti-slan Antikörper oder die Bindungseinheit bindet, erfolgt die Bindung so, dass die Funktionalität von Antikörper oder Bindungseinheit nicht beeinträchtigt ist. Damit ist gemeint, dass der anti-slan Antikörper nach der Bindung mit gleicher Affinität an slan-DC bindet bzw. dass die Bindungseinheit mit gleicher Affinität an den Co-Stimulus bindet.

Die weitere Bindungseinheit ist also so ausgestaltet, dass sie zur Bindung an den anti-slan Antikörper, an die einen Liganden bindende Bindungseinheit oder an das Antigen geeignet ist. Vorzugsweise ist die weitere Bindungseinheit ein Antikörper. Dieser Antikörper bindet nicht an slan-DC.

Vorzugsweise ist die weitere Bindungseinheit zur Bindung an die einen Liganden bindende Bindungseinheit geeignet.

Besonders bevorzugte erfindungsgemäße Zusammensetzungen sind dadurch gekennzeichnet, dass die Bindungseinheit ein Peptid ist und die weitere Bindungseinheit ein Antikörper ist, der das Peptid spezifisch bindet. Davon weiter bevorzugte Peptide umfassen die folgende Aminosäuresequenz aufweist: EKEALKKIIEDQQESLNK (SEQ ID No. 23, entspricht den Aminosäuren Nr. 311 - Nr. 328 des humanen La-Proteins gemäß SEQ ID No. 25). Diese Peptide werden hierin auch als "E7B6" bezeichnet. In Kombination mit diesen Peptiden ist die weitere Bindungseinheit bevorzugt ein Antikörper (hierin auch als "7B6" bezeichnet), der die die folgenden CDR-Regionen umfasst und das Peptid mit SEQ ID No. 23 spezifisch bindet:
a) variable Region der schweren Kette (V_{H}): CDR1 SEQ ID No. 26, CDR2 SEQ ID No. 27, CDR3 SEQ ID No. 28 und
b) variable Region der leichten Kette (V_{L}): CDR1 SEQ ID No. 29, CDR2 SEQ ID No. 30, CDR3 SEQ ID No. 31.

Die weitere Bindungseinheit ist bevorzugt mit dem anti-slan Antikörper und/oder mit dem Antigen über eine kovalente Bindung verbunden. Die kovalente Bindung ist bevorzugt eine Peptidbindung.

Vorzugsweise ist als weitere Bindungseinheit ein Antikörper in einer erfindungsgemäßen Zusammensetzung enthalten.

Bevorzugt sind in erfindungsgemäßen Zusammensetzungen mindestens zwei Fusionsproteine enthalten, von denen eines das Antigen und eines den anti-slan Antikörper enthält. Beide Fusionsproteine sind so ausgestaltet, dass sie miteinander einen Komplex bilden, d.h. dass jeweils eine Domäne eines Fusionsproteins mit einer Domäne des anderen Fusionsproteins eine spezifische Bindung eingeht. Diese Bindung ist bevorzugt eine Antikörper-Antigen-Bindung. In diesem Aufbau dient das Fusionsprotein, welches den anti-slan Antikörper enthält, zur Bindung an slan-DC. Dadurch, dass der Komplex das weitere Fusionsprotein mit dem Antigen enthält, wird das Antigen spezifisch an slan-DC transportiert. Dieser Aufbau eines Komplexes aus mindestens zwei Fusionsproteinen, die einander spezifisch binden, wird hierin auch als "modularer Aufbau" bezeichnet. Dadurch kann vorteilhaft mit einem Fusionsprotein, welches den anti-slan Antikörper enthält eine beliebige Vielzahl an Fusionsproteinen, welche Antigene enthalten, kombiniert werden.

Bevorzugt werden erfindungsgemäße Zusammensetzungen, bei denen entweder
a) der anti-slan Antikörper und die Bindungseinheit als Fusionsprotein vorliegen und die weitere Bindungseinheit und das Antigen als Fusionsprotein vorliegen oder
b) der anti-slan Antikörper und die weitere Bindungseinheit als Fusionsprotein vorliegen und das Antigen und die Bindungseinheit als Fusionsprotein vorliegen.

Dadurch, dass die Bindungseinheit und die weitere Bindungseinheit in unterschiedlichen Fusionsproteinen vorliegen und einander spezifisch binden, liegen die beiden Fusionsproteine in der erfindungsgemäßen Zusammensetzung als Komplex vor.

In einer bevorzugten Ausgestaltung der Erfindung nach a) enthält die erfindungsgemäße Zusammensetzung den anti-slan Antikörper und als Bindungseinheit ein Peptid in Form eines Fusionsproteins. Weiter enthält die erfindungsgemäße Zusammensetzung in diesem Fall ein Fusionsprotein, welches das Antigen und einen Antikörper enthält, der das Peptid spezifisch bindet. Das Peptid weist vorzugsweise eine alpha-Helix Struktur auf. Insbesondere umfasst das Peptid eine alpha-helikale Struktur, die durch die Aminosäuresequenz gemäß SEQ ID No. 23, insbesondere durch die Aminosäuresequenz gemäß SEQ ID No. 24 oder durch Aminosäuresequenzen mit einer Aminosäuresequenzidentität von mindestens 90 %, vorzugsweise mindestens 95% zu den genannten Sequenzen, gekennzeichnet ist. Besonders bevorzugt in dieser Ausgestaltung ist die Kombination eines Peptids E7B6 als Bindungseinheit mit einem Antikörper 7B6 als weitere Bindungseinheit. Als anti-slan Antikörper sind in diesen erfindungsgemäßen Zusammensetzungen die in Tabelle 2 dargestellten, gegebenenfalls humanisierten, Strukturen, bevorzugt, mit den Strukturen der Antikörper MB4 oder MB6.

In einer bevorzugten Ausgestaltung der Erfindung nach b) enthält die erfindungsgemäße Zusammensetzung den anti-slan Antikörper und als weitere Bindungseinheit einen Antikörper (hierin auch "weiterer Antikörper"), wobei anti-slan Antikörper und der weitere Antikörper in Form eines bispezifischen Antikörpers vorliegen. Bevorzugt liegen die Antikörper in Form eines single chain bispecific Diabodys (scBsDb) oder single chain bispecific Tandem Antikörpers (scBsTaFv) vor. Der weitere Antikörper ist nicht identisch mit dem anti-slan Antikörper und ist bindet nicht an slan-DC. Vorzugsweise bindet der weitere Antikörper spezifisch an eine strukturelle Domäne des weiteren Fusionsproteins der erfindungsgemäßen Zusammensetzung, welches das Antigen enthält. Bei Kombination des bispezifischen Antikörpers aus anti-slan Antikörper und weiterem Antikörper mit dem weiteren, das Antigen enthaltenden Fusionsprotein wird dann ein Komplex ausgebildet.

Die Avidität der anti-slan Antikörper wird dabei erhöht, wenn das weitere Fusionsprotein mehrere strukturelle Domänen enthält, die der weitere Antikörper bindet. Dies führt zur Bindung von großen Komplexen mit mehreren bispezifischen Antikörpern und zu einer verbesserten Bindung an slan-DC. Die strukturelle Domäne, die der weitere Antikörper bindet, ist vorzugsweise eine alpha-Helix Struktur, die bevorzugt in Form eines Linkerpeptids in dem weiteren Fusionsprotein vorliegt. Bevorzugt umfasst das weitere Fusionsprotein mehrere dieser Linkerpeptide, vorzugsweise mehr als 2, weiter bevorzugt 2 oder 3.

Besonders bevorzugt bindet der weitere Antikörper des erfindungsgemäßen Fusionsproteins eine alpha-helikale Peptidsequenz, die durch die Aminosäuresequenz gemäß SEQ ID No. 23, insbesondere durch die Aminosäuresequenz gemäß SEQ ID No. 24 oder durch Aminosäuresequenzen mit einer Aminosäuresequenzidentität von mindestens 90 %, vorzugsweise mindestens 95% zu den genannten Sequenzen, gekennzeichnet ist. Besonders bevorzugt in dieser Ausgestaltung ist die Kombination eines Peptids E7B6 als Bindungseinheit mit einem Antikörper 7B6 als weitere Bindungseinheit. Als anti-slan Antikörper sind in diesen erfindungsgemäßen Zusammensetzungen die in Tabelle 2 dargestellten, gegebenenfalls humanisierten, Strukturen, bevorzugt, mit den Strukturen der Antikörper MB4 oder MB6.

Die erfindungsgemäßen Zusammensetzungen eignen sich für eine therapeutische Anwendung, insbesondere im Menschen. Die Zusammensetzungen können dabei sowohl für Therapien eingesetzt werden, bei denen Toleranz gegen ein Antigen induziert werden soll als auch für Therapien, bei denen eine Immunantwort gegen ein Antigen induziert werden soll.

Die Erfindung umfasst weiter eine Nukleinsäuresequenz kodierend für einen erfindungsgemäßen anti-slan Antikörper.

Der Begriff "Nukleinsäuren" im Sinne der Erfindung umfasst neben Desoxyribonukleinsäuren (DNA) und Ribonukleinsäuren (RNA) auch alle anderen linearen Polymeren in denen die Basen Adenin (A), Cytosin (C), Guanin (G) und Thymin (T) oder Uracil (U) in entsprechender Abfolge angeordnet sind (Nukleinsäuresequenz). Die Erfindung umfasst dabei auch die korrespondierenden RNA-Sequenzen (in denen Thymin durch Uracil ersetzt ist), komplementäre Sequenzen und Sequenzen mit modifiziertem Nukleinsäurerückgrat oder 3' oder 5'-Terminus. Der Begriff "Nukleinsäuresequenzen mit verändertem Rückgrat" umfasst dabei alle anderen linearen Polymere in denen die Basen Adenin (A), Cytosin (C), Guanin (G) und Thymin (T) oder Uracil (U) in entsprechender Abfolge angeordnet sind, wie z. B. Sequenzen mit einem Phosphothioat-, Phosphoramidat- oder O-Methyl-derivatisierten Rückgrat, Peptid-Nukleinsäuren (PNA) und locked nucleic acids (LNA) oder gemischtem Rückgrat. Der Begriff "modifizierter 3' oder 5' Terminus" umfasst dabei sowohl Modifikationen, die der Stabilisierung dienen als auch das Anbinden von Markern. Beispiele für Marker sind Enzyme, Farbstoffe oder Fluoreszenzfarbstoffe, Radionukleotide, sowie Haptene, wie z. B. Digoxigenin oder Biotin.

Bevorzugt umfasst die Nukleinsäuresequenz, die für den erfindungsgemäßen Antikörper MB4 kodiert, die folgenden Nukleinsäuresequenzen: variable Region der schweren Kette SEQ ID No. 11 und variable Region der leichten Kette SEQ ID No. 13. Bevorzugt umfasst die Nukleinsäuresequenz, die für den erfindungsgemäßen Antikörper MB6 kodiert, die folgenden Nukleinsäuresequenzen: variable Region der schweren Kette SEQ ID No. 15 und variable Region der leichten Kette SEQ ID No. 17.

Weiter umfasst die Erfindung einen Expressionsvektor, der eine erfindungsgemäße Nukleinsäuresequenz enthält. Im Sinne der Erfindung wird unter einem Expressionsvektor ein Plasmid, Virus oder anderer Träger verstanden, der eine erfindungsgemäße Nukleinsäuresequenz rekombinant durch Insertion oder Inkorporation enthält. Der Expressionsvektor enthält typischerweise einen Replikationsstartpunkt, einen Promoter, sowie spezifische Gensequenzen, die eine phänotypische Selektion von den Expressionsvektor enthaltenden Wirtszellen ermöglichen.

Weiter umfasst die Erfindung eine Wirtszelle oder einen nicht-menschlicher Wirtsorganismus, welche rekombinant eine erfindungsgemäße Nukleinsäuresequenz oder einen erfindungsgemäßen Expressionsvektor enthalten.

Eine Wirtszelle ist eine natürlich vorkommende Zelle oder eine transformiert oder genetisch veränderte Zelllinie, welche mindestens einen erfindungsgemäßen Expressionsvektor enthält. Die Erfindung umfasst dabei transiente Transfektanten (z. B. durch mRNA-Injektion) oder Wirtszellen, in denen mindestens ein erfindungsgemäßer Expressionsvektor als Plasmid oder künstliches Chromosom enthalten ist, sowie Wirtszellen in denen ein erfindungsgemäßer Expressionsvektor stabil in das Genom des Wirtes integriert ist. Die Wirtszelle ist bevorzugt ausgewählt aus Prokaryonten oder Eukaryonten. Bevorzugte Prokaryonten sind ausgewählt aus Escherichia coli, Bacillus subtilis.

Bevorzugte Eukaryonten sind Hefezellen (z. B. Saccharomyces cerevisiae, Pichia pastoris), Insektenzellen, amphibische Zellen oder Säugetierzellen, wie z. B. CHO, HeLa, HEK293.

Nicht-menschliche Wirtsorganismen enthalten einen erfindungsgemäßen Expressionsvektor, welcher stabil in das Genom des Wirtsorganismus oder einzelner Zellen des Wirtsorganismus integriert ist. Bevorzugte Wirtsorganismen sind Pflanzen, Invertebraten oder Vertebraten, insbesondere Bovidae, Drosophila melanogaster, Caenorhabditis elegans, Xenopus laevis, Medaka, Zebrafisch oder Mus musculus, oder Zellen oder Embryonen der genannten Organismen.

Die Herstellung eines erfindungsgemäßen anti-slan Antikörpers erfolgt bevorzugt durch ein Verfahren, bei dem man:
a) eine erfindungsgemäße Wirtszelle oder einen erfindungsgemäßen nicht-menschlichen Wirtsorganismus Bedingungen aussetzt unter denen eine Expression und gegebenenfalls eine Sekretion des anti-slan Antikörpers stattfindet und gegebenenfalls
b) den anti-slan Antikörper wenigstens teilweise aufreinigt.

Bevorzugt werden dafür erfindungsgemäße Wirtszellen in einem Selektivmedium gezüchtet (kultiviert), das auf das Wachstum der Zellen selektiert, die einen erfindungsgemäßen Expressionsvektor enthalten. Die Expression der Gensequenzen des Expressionsvektors resultiert in der Produktion des erfindungsgemäßen anti-slan Antikörpers. Die exprimierten anti-slan Antikörper werden dann vorzugsweise durch ein beliebiges konventionelles Verfahren isoliert und gereinigt, einschließlich Extraktion, Präzipitation, Chromatographie, Elektrophorese oder auch durch Affinitätschromatographie.

Erfindungsgemäße Zusammensetzungen eignen sich zur therapeutischen Anwendung bei der Behandlung oder Prophylaxe von Krankheiten, wobei entweder antigenspezifisch eine Immunantwort vermittelt oder Toleranz induziert wird. Dabei werden die jeweiligen Antigene mit dem ggf. erforderlichen (vorzugsweise aktivierenden) Co-Stimulus über die anti-slan Antikörper oder daraus gebildete Komplexe an slan-DC transportiert.

Krankheiten, bei denen antigenspezifisch eine Immunantwort ausgelöst werden soll, sind insbesondere Tumorerkrankungen oder Infektionen. Dabei werden zur Therapie erfindungsgemäße Zusammensetzungen in Kombination mit einem Co-Stimulus eingesetzt, der slan-DC aktiviert. Bevorzugt ist der aktivierende Co-Stimulus direkt an einen Bestandteil der erfindungsgemäßen Zusammensetzung gebunden und wird so nach Verabreichung gezielt an slan-DC transportiert. Bevorzugte aktivierende Co-Stimuli sind Liganden von PRR, insbesondere von TLR. Insbesondere werden in Kombination mit erfindungsgemäßen Zusammensetzungen TLR-Liganden eingesetzt, bevorzugt ssRNA als Ligand von TLR7 oder TLR8, dsRNA als Ligand von TLR3 oder unmethylierte bakterielle DNA (CpG-DNA) als Ligand von TLR9.

Dadurch wird das in der Zusammensetzung enthaltene Antigen direkt an slan-DC transportiert und nach Bindung des anti-slan Antikörpers an die Zelloberfläche von den slan-DC prozessiert und auf deren Oberfläche präsentiert. Gleichzeitig wird die Zelle durch den Co-Stimulus aktiviert. Durch die Antigenpräsentation der aktivierten slan-DC wird eine pathogene Immunreaktion gegen das Antigen vermittelt. Antigene, die bei dieser Therapievariante bevorzugt in den erfindungsgemäßen Zusammensetzungen enthalten sind, sind Tumorantigene (zur Behandlung von Tumorerkrankungen) oder Antigene von Pathogenen (zur Behandlung von Infektionen). Besonders bevorzugt sind dabei die obengenannten Tumorantigene.

Die Erfindung umfasst ebenfalls einen pharmazeutischen Kit, welcher eine erfindungsgemäße Zusammensetzung und einen Liganden, der die Aktivierung von humanen dendritischen Zellen, insbesondere die Aktivierung von slan-DC, bewirkt und an mindestens einen Bestandteil der erfindungsgemäßen Zusammensetzung bindet in Assoziation mit einem pharmazeutisch annehmbaren Verdünnungsmittel oder Träger.

Bevorzugt umfasst der pharmazeutische Kit in Assoziation mit einem pharmazeutisch annehmbaren Verdünnungsmittel oder Träger:
a) eine erfindungsgemäße Zusammensetzung umfassend:
   - einen bispezifischen Antikörper, enthaltend MB4 oder MB6 als anti-slan Antikörper und einen 7B6 Antikörper,
   - ein Fusionsprotein, enthaltend ein Peptid gemäß SEQ ID No. 23 (E7B6) und ein Tumorantigen, und
b) mindestens einen TLR-Liganden ausgewählt aus ssRNA und CpG-DNA.

Vorzugsweise liegen die Antikörper im erfindungsgemäßen pharmazeutischen Kit als chimäre oder besonders bevorzugt humanisierte Antikörper vor, die eine verringerte Immunogenität aufweisen.

Die erfindungsgemäßen pharmazeutischen Zusammensetzungen umfassen verschiedene Dosierungsformen. Die pharmazeutischen Zusammensetzungen werden bevorzugt parenteral, besonders bevorzugt intravenös verabreicht. In einer Ausgestaltung der Erfindung liegt die parenterale pharmazeutische Zusammensetzung in einer Darreichungsform vor, die zur Injektion geeignet ist. Besonders bevorzugte pharmazeutische Zusammensetzungen sind daher Lösungen, Emulsionen oder Suspensionen des Antikörpers welches in einem pharmazeutisch annehmbaren Verdünnungsmittel oder Träger vorliegt.

Als Träger werden vorzugsweise Wasser, gepuffertes Wasser, 0,4% Salzlösung, 0,3% Glycin und ähnliche Lösungsmittel eingesetzt. Die Lösungen sind steril. Die pharmazeutischen Zusammensetzungen werden durch herkömmliche, gut bekannte Techniken sterilisiert. Die Zusammensetzungen enthalten bevorzugt pharmazeutisch akzeptable Hilfssubstanzen, wie etwa diejenigen, die erforderlich sind um näherungsweise physiologische Bedingungen zu ergeben und/oder die Stabilität der in der Zusammensetzung enthaltenen Antikörper zu erhöhen, wie etwa Mittel zur Einstellung des pH-Werts und Puffermittel, Mittel zur Einstellung der Toxizität und dergleichen, vorzugsweise ausgewählt aus Natriumacetat, Natriumchlorid, Kaliumchlorid, Calciumchlorid und Natriumlactat.

Bevorzugt ist die pharmazeutische Zusammensetzung eine injizierbare gepufferte Lösung, die zwischen 0,1 bis 500 mg/ml Antikörper, besonders bevorzugt zwischen 0,1 bis 250 mg/ml Antikörper, insbesondere zusammen mit 1 bis 500 mMol/l Puffer enthält. Die injizierbare Lösung kann sowohl in einer flüssigen als auch ein einer lyophilisierten Dosierungsform vorliegen. Der Puffer ist bevorzugt ausgewählt aus Histidin, Natriumsuccinat, Natriumcitrat, Natriumphosphat und Kaliumphosphat.

Die Erfindung umfasst auch die Verwendung eines erfindungsgemäßen pharmazeutischen Kits oder einer erfindungsgemäßen Zusammensetzung zur therapeutischen Behandlung von Tumorerkrankungen. Das Antigen, welches in der erfindungsgemäßen Zusammensetzung enthalten ist, ist in diesem Fall ein Tumorantigen. Für therapeutische Anwendungen wird eine sterile erfindungsgemäße Zusammensetzung oder eine sterile erfindungsgemäße pharmazeutische Zusammensetzung, welche eine pharmakologisch wirksame Dosismenge der Antikörper enthält, einem Patienten verabreicht, um Tumorerkrankungen zu behandeln.

Krankheiten, bei denen antigenspezifisch Toleranz induziert werden soll, sind insbesondere Allergien Autoimmunerkrankungen oder Transplantatabstoßungsreaktionen durch eine Host versus Graft Reaktion bzw. eine Verhinderung einer Graft versus Host Reaktion. Bei der therapeutischen Behandlung von Allergieren sind die Antigene Allergene, bei der therapeutischen Behandlung von Autoimmunkrankheiten sind die Antigene Autoantigene. Bei Transplantatabstoßungsreaktionen sind die Antigene insbesondere Abweichungen in den "major" und "minor" Histokompatibilitätsantigenen zwischen Spender und Empfänger. Es werden zur Therapie dieser Erkrankungen erfindungsgemäße Zusammensetzungen ohne zusätzlichen Co-Stimulus verabreicht, so dass slan-DC nicht aktiviert werden. Dadurch wird das in der erfindungsgemäßen Zusammensetzung enthaltene Antigen von den slan-DC prozessiert und auf deren Oberfläche präsentiert. Durch die Antigenpräsentation von nicht aktivierten slan-DC wird eine Hemmung pathogener Immunreaktionen auf das Antigen (Toleranzinduktion gegen das Antigen) vermittelt und so die Symptome der Erkrankung gemildert.

Erfindungsgemäße Zusammensetzungen sind vorteilhaft für die Therapie von Tumorerkrankungen geeignet. Dadurch, dass Liganden, die dendritische Zellen (DC) aktivieren können, gezielt zu DC transportiert werden können, ist mit erfindungsgemäßen Zusammensetzungen keine systemische Applikation dieser Liganden notwendig. Die Liganden werden speziell zu den DC transportiert und können auch nur dort wirken. Dies verhindert, dass durch diese Liganden, die üblicherweise auch Rezeptoren auf anderen Zellen, insbesondere auf Zellen des Immunsystems binden können, andere Zellen aktiviert und unerwünschte Reaktionen vermittelt werden. Die erfindungsgemäßen Antikörper eignen sich besonders vorteilhaft für die Anwendung in erfindungsgemäßen Zusammensetzungen, da sie selbst in der Form von scFv-Fragmenten eine höhere Bindungsaffinität zu slan-DC als die bekannten Antikörper DD2 und MDC-8 aufweisen.

Sie sind darüberhinaus auch anderen bekannten Oberflächenantigenen auf DC überlegen, die zum Targeting von DC genutzt werden, insbesondere DEC-205. Dies liegt daran, dass das slan-Epitop selektiv auf slan-DC exprimiert wird, wohingegen DEC-205 auch auf anderen Geweben, z.B. epithelialen Zellen im Thymus (Nonaka et al., J. Am J Surg Pathol. 2007, 31:1038-44), exprimiert wird. Darüber hinaus wird das slan-Epitop bereits auf unreifen DCs, also vor der Aufnahme von Antigen, exprimiert, wohingegen DEC-205 z.B. auf Langerhans Zellen (unreifen DCs) in der Haut herunterreguliert ist und erst nach Reifung (also nach der Aufnahme und Aktivierung durch das Antigen bzw. Cofaktoren) auf dem Weg in bzw. im Lymphknoten hochreguliert wird (Ebner et al., Int. Immunol. 2004, 16, 877-87).

Anhand folgender Figuren und Ausführungsbeispiele soll die Erfindung näher erläutert werden, ohne die Erfindung auf diese zu beschränken.

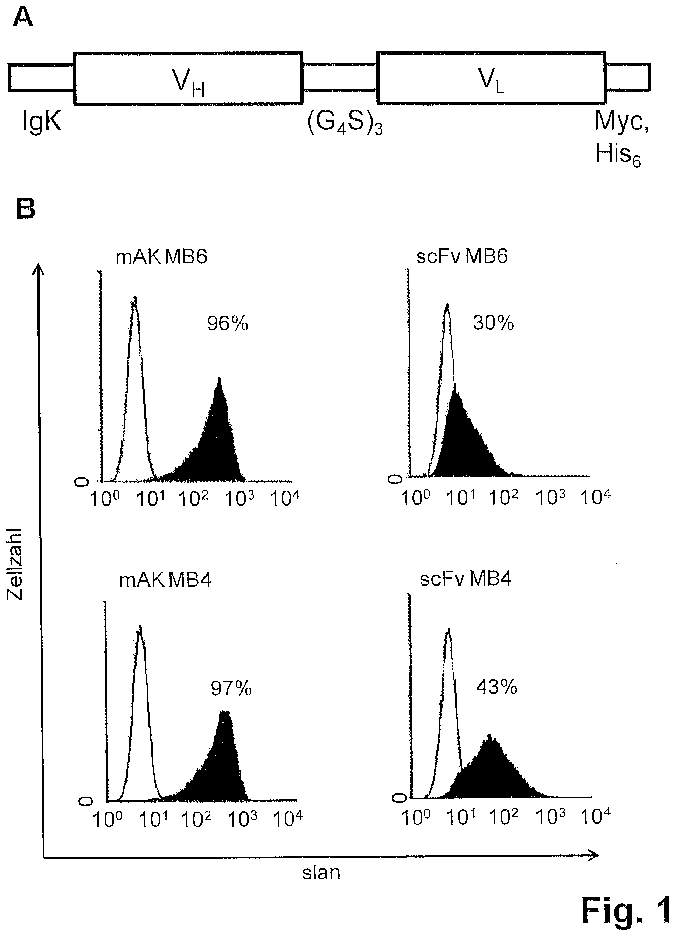

- Fig. 1: (A) Schematischer Aufbau von scFv-Fragmenten, die die variablen Regionen der schweren und leichten Kette der erfindungsgemäßen Antikörper MB4 bzw. MB6 enthalten; (B) Bindung der erfindungsgemäßen anti-slan Antikörper MB4 (untere Reihe) und MB6 (obere Reihe) an slan-DC. MB4 und MB6 wurden in der Form monoklonaler IgM Antikörper (linke Reihe) und in der Form eines scFv-Fragments (rechte Reihe) getestet.

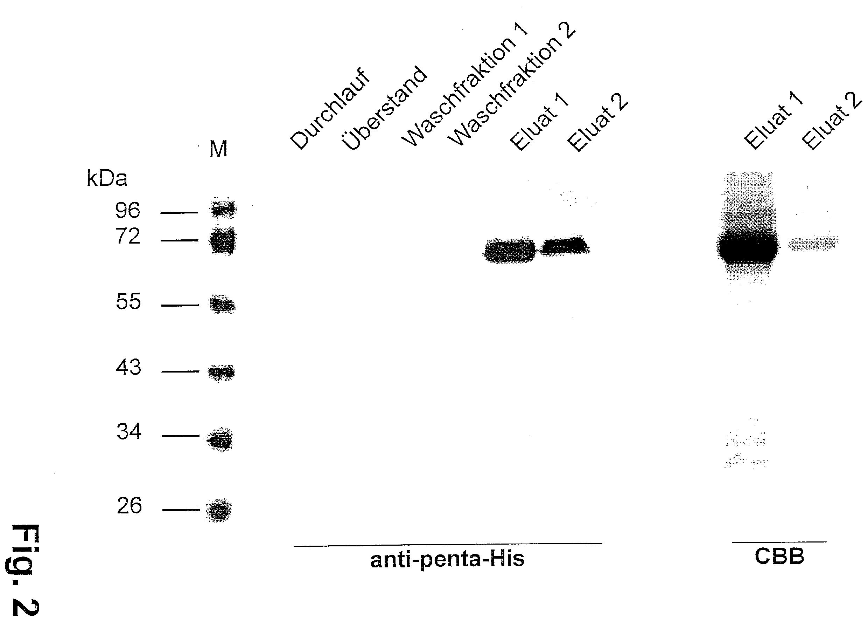

- Fig. 2: Immunoblot (A) und SDS-Page (B) von rekombinant hergestellten bispezifischen Antikörpern in Form von scBsDb mit einem anti-slan DD2 und einem 7B6-Antikörper (hierin [DD2-7B6]) nach Aufreinigung. Die eluierten Fraktionen ("elution 1", "elution 2") enthalten [DD2-7B6]. Bezeichnungen: M... Marker, supernatant ... Zellkulturüberstand, flow trough ... Durchlauf der Säule, wash 1, 2 ... Waschfraktion, anti-penta-HIS ... monoklonaler anti-penta-HIS Antikörper (Qiagen, Hilden, Deutschland), CBB ... Coomassie Billantblau.

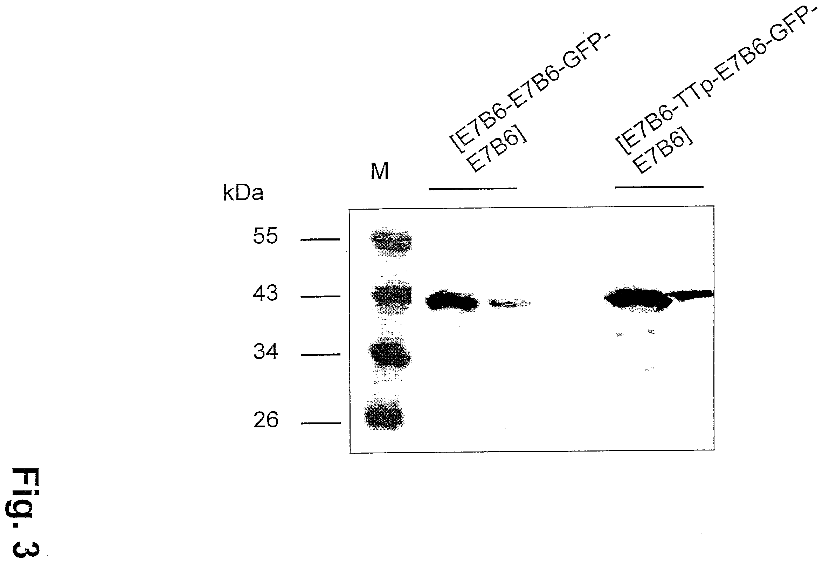

- Fig. 3: Immunoblot von Fusionsproteinen aus drei Bindungseinheiten (E7B6) und mindestens einem Antigen (GFP, TTp) nach Aufreinigung. Es wurden die Fusionsproteine [E7B6-E7B6-GFP-E7B6] (Aminosäuresequenz gemäß SEQ ID No. 37) und [E7B6-TTp-E7B6-GFP-E7B6] (Aminosäuresequenz gemäß SEQ ID No. 35) analysiert.

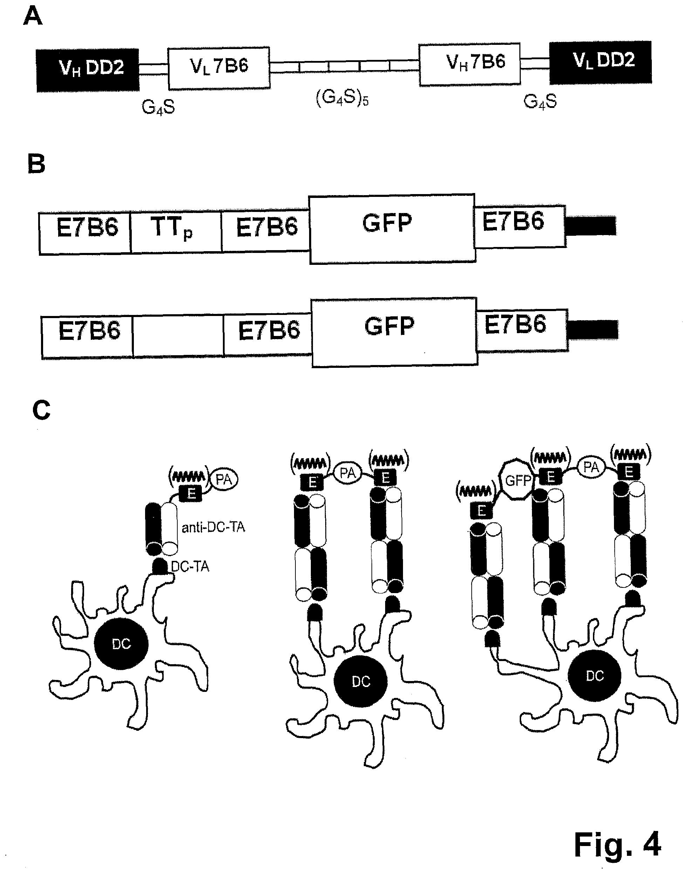

- Fig. 4: (A) Schematische Darstellung des Fusionsproteins aus Ausführungsbeispiel 2 [DD2-7B6] gemäß SEQ ID No. 33; (B) schematische Darstellung der Konstrukte aus Ausführungsbeispiel 3 [E7B6-E7B6-GFP-E7B6] (oberes Schema, Aminosäuresequenz gemäß SEQ ID No. 37) und [E7B6-TTp-E7B6-GFP-E7B6] (unteres Schema, Aminosäuresequenz gemäß SEQ ID No. 35); (C) schematische Darstellung des Wirkmechanismus der therapeutischen Anwendung erfindungsgemäßer Zusammensetzungen. Abkürzungen: DC ... slan dendritische Zelle (slan-DC), DC-TA ... slan-Epitop, anti-DC-TA ... Antikörper, der gegen das slan-Epitop gerichtet ist, E ... Bindungseinheit, die zur Bindung von DC aktivierenden Liganden (durch die stilisierte Nukleinsäurehelix gekennzeichnet) geeignet ist, PA ... Antigen. Antigene werden durch Fusionsproteine, die anti-slan Antikörper, die Bindungseinheit und das Antigen enthalten an slan-DC transportiert (C, linke Alternative). Antigene werden durch Komplexe aus Fusionsproteinen zu DC transportiert, wobei ein Fusionsprotein das Antigen enthält und ein weiteres Fusionsprotein den anti-slan Antikörper enthält. Beide Fusionsproteine binden einander (hier dargestellt als Antikörper-Bindung eines Antikörpers, der spezifisch an die Bindungseinheit E bindet).

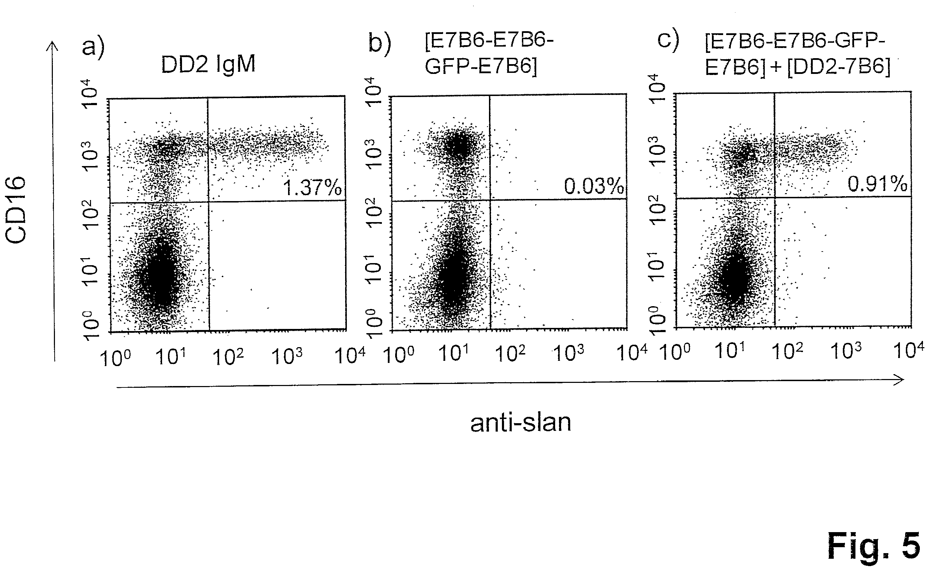

- Fig. 5: Bindung einer erfindungsgemäßen Zusammensetzung an native humane CD16^{+/}slan⁺ slan-DC. (A) Kontrolle: Anteil der slan-DC an nativen PBMC, nachgewiesen mit einem monoklonalen anti-slan IgM (DD2); (B) Kontrolle: [E7B6-E7B6-GFP-E7B6] (Aminosäuresequenz gemäß SEQ ID No. 37) allein ohne anti-slan Antikörper; (C) erfindungsgemäße Zusammensetzung enthaltend ein Fusionsprotein mit anti-slan Antikörper [DD2-E7B6] (gemäß SEQ ID No. 33) und ein Fusionsprotein mit Antigen [E7B6-E7B6-GFP-E7B6] (Aminosäuresequenz gemäß SEQ ID No. 37), wobei die Fusionsproteine als Komplex vorliegen, da sie durch die Bindung von 7B6 and E7B6 aneinander gebunden sind (MFI ... mittlere Fluoreszenzintensität).

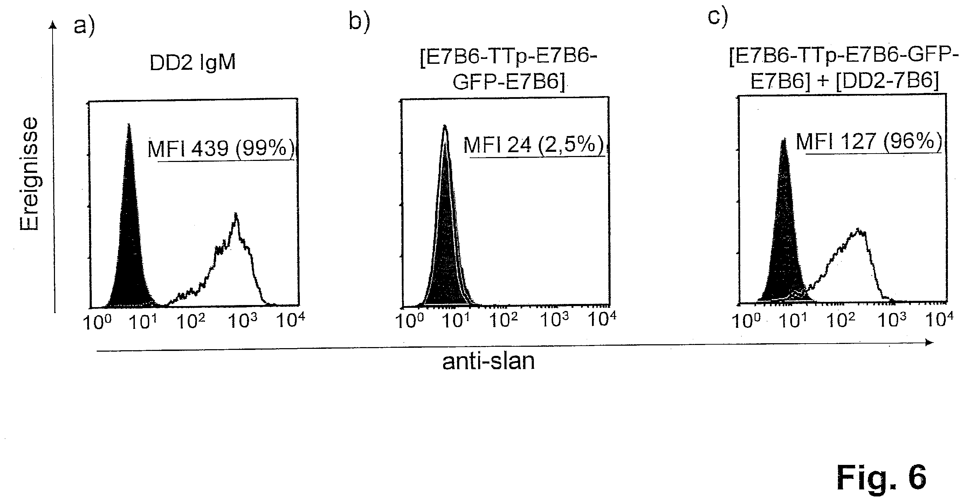

- Fig. 6: Bindung einer erfindungsgemäßen Zusammensetzung an slan-positive Jurkat-Zellen. (A) Kontrolle: Bindung von monoklonalem anti-slan IgM DD2; (B) Kontrolle: Bindung von [E7B6-TTp-E7B6-GFP-E7B6] (Aminosäuresequenz gemäß SEQ ID No. 35) ohne anti-slan Antikörper; (C) erfindungsgemäße Zusammensetzung enthaltend ein Fusionsprotein mit anti-slan Antikörper [DD2-E7B6] (gemäß SEQ ID No. 33) und ein Fusionsprotein mit zwei Antigenen [E7B6-TTp-E7B6-GFP-E7B6] (gemäß SEQ ID No. 35), wobei die Fusionsproteine als Komplex vorliegen, da sie durch die Bindung von 7B6 and E7B6 aneinander gebunden sind (MFI ... mittlere Fluoreszenzintensität).

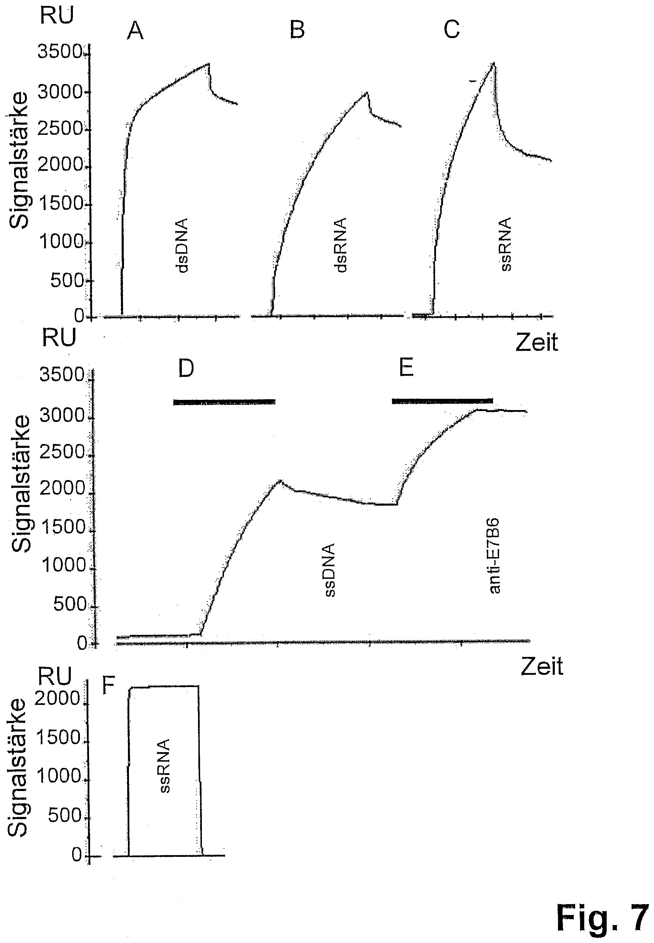

- Fig. 7: (A-F) Komplexbildung mit Nukleinsäuren durch erfindungsgemäße Fusionsproteine mit einem Peptid gemäß SEQ ID No. 23 (E7B6) bestimmt durch Komplexbildung an SPR-Chips. Komplexbildung mit (A) dsDNA, (B) dsRNA, (C) ssRNA, (D) ssDNA. (E) Bindung eines spezifischen Antikörpers nach Komplexbildung mit Nukleinsäure. (F) Vergleichsbeispiel: Bindung eines Fusionsproteins mit Gly-Ser-Linker an ssRNA. Figurenbeschriftung: Response ... Intensität der Bindung in RU (response units, Anbindungseinheiten), time ... Zeit.

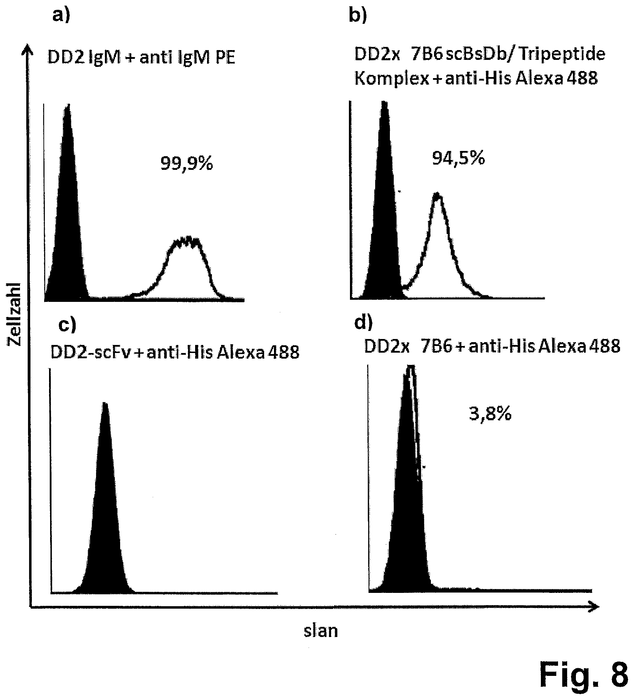

- Fig. 8: Bindung des scFv von DD2 an slan-positive Jurkat-Zellen ermittelt per FACS-Analyse. (a) Kontrolle: Bindung des monoklonalen IgM Antikörpers DD2, (b) Bindung einer erfindungsgemäßen Zusammensetzung gemäß Ausführungsbeispiel 2 und 3 ([DD2-7B6] und [E7B6-E7B6-GFP-E7B6]; in der Abbildung als "DD2x7B6 scBsDb/Tripeptide Komplex" bezeichnet), (c) Bindung des DD2 scFv Fragments, (e) Kontrolle: Bindung des bispezifischen Antikörpers aus Ausführungsbeispiel 2 [DD2-7B6].

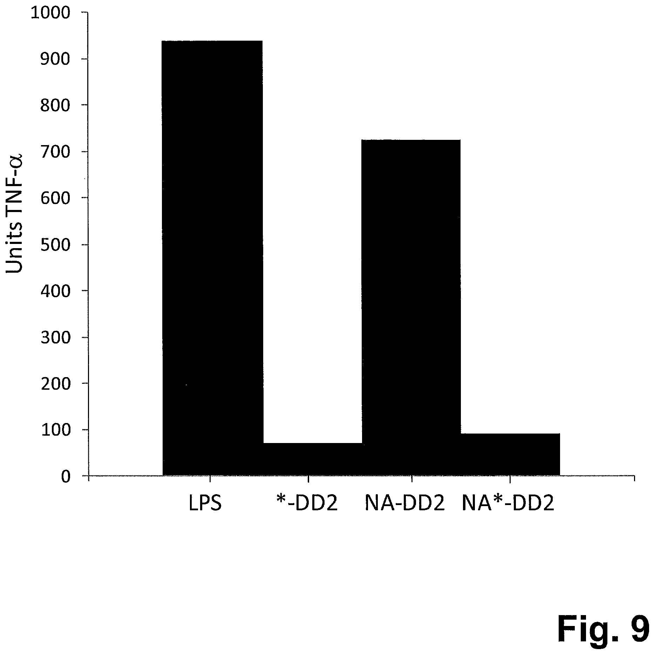

- Fig. 9: TNFa-Produktion von slan-DC nach TLR-abhängiger Aktivierung *in vitro* ermittelt im TNFα-ELISA. Frisch isolierte slan-DC wurden mit folgenden Proben versetzt: LPS (Positivkontrolle), *-DD2 (Negativkontrolle), NA-DD2 (erfindungsgemäße Zusammensetzung enthaltend mit Nukleinsäure aus *E.coli* beladenes Oligopeptid [E7B6-E7B6-GFP-E7B6] und [DD2-7B6]) sowie NA*-DD2 (Negativkontrolle).

### Ausführungsbeispiel 1: Bereitstellung von erfindungsgemäßen Antikörpern MB4 und MB6

Für die Generierung und das Screening der jeweiligen Hybridomüberstände der anti-slan Antikörper MB4 und MB6 wurden slan-DCs aus frisch isolierten peripheren Blutlymphozyten Präparationen (PBMCs) mittels magnetischer Zellseparation (MACS) isoliert.

Zur Isolation der slan-DC wurde Zellkulturüberstand enthaltend den monoklonalen IgM Antikörper M-DC8 im Verhältnis 1:60 in PBS/2 mM EDTA verdünnt. Je 10⁹ periphäre kernhaltige Zellen werden mit 20 ml verdünntem Zellkulturhybridomaüberstand für 15 min inkubiert. Nach der Inkubation werden die Zellen durch Zentrifugation gesammelt und mit 50 ml PBS/EDTA gewaschen. Die markierten Zellen werden mit anti-IgM Magnetbeads der Firma Miltenyi (Bergisch Gladbach) isoliert. Je 10⁹ periphäre kernhaltige Zellen werden in 1 ml PBS/EDTA zusammen mit 120 µl anti-IgM beads resuspendiert und für 15 min inkubiert. Danach werden die Zellen mit 50 ml PBS/ETDA gewaschen. Je 10⁹ Zellen wurden vor der Separation per MACS in 5 ml PBS/EDTA resuspendiert.

Derartig isolierte slan-DCs wurden zur Immunisierung von Mäusen verwendet. Pro Immunisierung und pro Maus wurden 5x10⁵ bis 1x10⁶ Zellen intravenös appliziert. Die Mäuse wurden vor der Hybridomafusion mindestens viermal immunisiert. Zur Hybridomafusion wurden Milzzellen der immunisierten Mäuse wurden mit Myelomzellen (P3 x Ag 8.653; ATCC CRL 1580) im Verhältnis 1:1 bis 10:1 durch tropfenweise Zugabe von Polyethylenglykol fusioniert. Die Zellen wurden in Zellkulturmedium (RPMI 1640) enthaltend 10% FCS und 300 Einheiten/ml humanes rekombinantes Interleukin 6 (Biochrom, Berlin) verdünnt und auf 96 well Zellkulturplatten ausgesät. Anschließend wurden die Zellen unter HAT-Medium (Medium enthaltend zusätzlich Hypoxanthin, Aminopterin und Thymidin) selektioniert. Positive Hybridome wurden mittels FACS Analyse von Hybridomaüberständen auf PBMCs identifiziert und die gegen slan-DC reaktiven Hybridomazellen wurden durch limitierte Verdünnung mehrfach rekloniert. Die Hybridome MB4 und MB6 gingen aus einer Vielzahl von über 100 Fusionen hervor.

Auf Basis der monoklonalen IgM Antikörper MB4 und MB6 wurden scFv-Fragmente kloniert, die schematisch in Fig. 1A dargestellt sind. In den scFv-Fragmenten sind die variablen Domänen der monoklonalen Antikörper MB4 bzw. MB6 in der Reihenfolge "V_{H}-V_{L}" über einen flexiblen Glycin-Serin-Linker (in der Abbildung mit (G₄S)₃ bezeichnet) miteinander verknüpft. Die Konstrukte wurden in einen Vektor kloniert, der ebenfalls für eine Signalsequenz kodiert, die die Sezernierung der Antikörper aus der Zelle bewirkt. Weiterhin enthalten die Konstrukte C-terminal ein c-myc-Tag (zur Markierung) und ein Hexahistidin-(His₆)-Tag (zur Aufreinigung des Konstrukts).

Die Vektoren wurden mit Lipofectamin 2000 (Invitrogen, Karlsruhe, Deutschland) transient in HEK293T Zellen transfiziert. Nach der Aufreinigung der scFvs von MB4 und MB6 aus dem Zellkulturüberstand durch Nickelaffinitätschromatographie wurden die scFv und die monoklonalen Antikörper per FACS-Analyse auf ihre Bindung an native slan-DC getestet. Die Detektion der scFv erfolgte mit anti-c-myc-FITC. Die Detektion der monoklonalen IgM-Antikörper erfolgte mit anti-Maus-IgM-Alexa Fluor® 488 (Invitrogen, Karlsruhe, Deutschland).

Die FACS-Analyse (Fig. 1B) zeigt, dass sowohl die monoklonalen anti-slan Antikörper MB6 und MB4 als auch die aus deren variablen Domänen der schweren und leichten Ketten konstruierten scFvs an slan-DCs binden.

Die Affinität der monoklonalen Antikörper MB4 und MB6 gegenüber slan DC beträgt 0,4x10⁻¹² mol/l und ist damit ca. 25-fach höher als die Affinität der parallel bestimmten monoklonalen anti-slan Antikörper DD2 bzw. M-DC8.

### Ausführungsbeispiel 2: Bereitstellung eines Fusionsproteins in Form eines bispezifischen Antikörpers, enthaltend einen anti-slan Antikörper (DD2)

Zur Bereitstellung einer erfindungsgemäßen Zusammensetzung wurde zunächst ein Fusionsprotein hergestellt, welches einen anti-slan Antikörper enthält. Im Ausführungsbeispiel 2 wurde dafür ein Fusionsprotein in Form eines single chain bispecific Diabodys (scBsDb) gewählt, welches als anti-slan Antikörper ein scFv-Fragment des monoklonalen anti-slan IgM DD2 enthält und einen weiteren Antikörper, nämlich ein scFv-Fragment des 7B6 Antikörpers enthält. Diese scBsDb werden hierin auch schematisch als [DD2-7B6] abgekürzt und umfassen eine Aminosäuresequenz gemäß SEQ ID No. 33. Eine Nukleinsäuresequenz, die für [DD2-7B6] kodiert, ist in SEQ ID No. 32 genannt.

Der 7B6 Antikörper bindet spezifisch eine Peptidstruktur gemäß SEQ ID No. 23 und eignet sich daher zur Komplexbildung der Fusionsproteine mit Fusionsproteinen die die Peptidstruktur gemäß SEQ ID No. 23 enthalten. Ein Beispiel dafür wird in Ausführungsbeispiel 3 genannt.

Zur Klonierung der [DD2-7B6] wurden die cDNA-Sequenzen der schweren und leichten Kette von DD2 und 7B6 in Form isoliert. Dazu wurden RNA aus den jeweiligen Hybridomen isoliert (TriPure Reagenz von Roche, Mannheim, Deutschland) und mit dem Advantage RT-for-PCR Kit (Clontech/TaKaRa, Saint-Germain-en-Laye, France) in cDNA umgewandelt. Die jeweiligen cDNAs wurden mit Hilfe des Advantage-HF 2 PCR Kit (Clontech/TaKaRa, Saint-Germain-en-Laye, France) gemäß Wang et al. (J. Immunol. Methods 2000, 13, 167-77) amplifiziert. Zur Amplifikation der schweren Ketten des anti-slan DD2 Antikörpers wurden die Primer gemäß SEQ ID No. 38 und SEQ ID No. 39 verwendet. Zur Amplifikation der schweren Ketten des 7B6 Antikörpers wurden die Primer gemäß SEQ ID No. 38 und SEQ ID No. 40 verwendet. Die Amplifikation der leichten Ketten erfolgte für beide Antikörper unter Verwendung der Primer gemäß SEQ ID No. 41 und SEQ ID No. 42. Die entstandenen PCR-Produkte wurden in einen pGEM-T easy Vektor (Promega, Mannheim, Germany) kloniert und sequenziert. Die pGEM-T Klone wurden als Substrat für SOE PCR zur Herstellung des DD2-scFv-Fragmentes verwendet, bei der die Primer gemäß SEQ IDs No. 43 bis 46 eingesetzt wurden. Für die Herstellung des 7B6-scFv-Fragmentes wurden die Primer gemäß SEQ IDs No. 47 bis 50 eingesetzt.

Um das scBsDb [DD2-7B6] zu klonieren, wurden die schwere und leichte Kette des 7B6 scFv in die Reihenfolge "leichte Kette/schwere Kette" umkloniert. Dadurch wurde die schwere Kette von 7B6 mit den Primern gemäß SEQ IDs No. 51 und 52 amplifiziert und die leichte Kette von 7B6 mit den Primern gemäß SEQ IDs No. 53 und 54 amplifiziert. Die leichte Kette wurde upstream der schweren Kette von 7B6 kloniert. Das 7B6 "leichte Kette/schwere Kette" Konstrukt wurde dann in das DD2 scFv kloniert, und der scBsDb [DD2-7B6] gebildet.

Zu Expression der rekombinanten Antikörper wurden alle Leserahmen der scFv und auch die Leserahmen des scBsDb [DD2-7B6] in die Vektoren pSEC-Tag2B und pcz CFG 5.1 kloniert. Dafür wurden per PCT künstliche Schnittstellen für Restriktionsenzyme (entweder SfiI und Notl oder EcoRI und Kpn2I) eingefügt. Die gebildeten Konstrukte im pSEC-Tag2B wurden mit Lipofectamin 2000 (Invitrogen, Karlsruhe, Deutschland) transient in HEK293T Zellen transfiziert. Die gebildeten Konstrukte im pcz CFG 5.1 wurden zur Herstellung stabil transduzierter Zellinien verwendet. Die rekombinanten scFv (7B6 scFv oder DD2 scFv) oder scBsDb [DD2-7B6] wurden aus dem Zellkulturüberstand mit Hilfe von Affinitätschromatographie über Ni-NTA Agarose (Qiagen, Hilden, Deutschland) aufgereinigt. Per SDS-Page und Immunoblot wurde die Reinheit und Stabilität des scBsDb [DD2-7B6] nachgewiesen (Fig. 2).

### Ausführungsbeispiel 3: Bereitstellung eines Fusionsproteins enthaltend eine Bindungseinheit (Peptid E7B6) und ein Antigen (ein antigenes Peptid aus Tetanustoxin)

Als weiterer Bestandteil einer erfindungsgemäßen Zusammensetzung, der hier beispielhaft in Kombination mit dem Fusionsprotein aus Ausführungsbeispiel 2 eingesetzt wird, wurde ein Fusionsprotein hergestellt, welches als Bindungseinheiten ein Peptid mit der Aminosäuresequenz gemäß SEQ ID No. 23 (E7B6) enthält und als Antigen das grün fluoreszierende Protein (GFP) enthält. Diese Konstrukte werden hierin auch mit [E7B6-E7B6-GFP-E7B6] (Aminosäuresequenz gemäß SEQ ID No. 37, Nukleinsäuresequenz gemäß SEQ ID No. 36) abgekürzt. Dies bedeutet, dass das Peptid E7B6 dreimal im Konstrukt vorliegt. GFP diente zusätzlich als Marker und als Spacermolekül.

Weitere Konstrukte enthalten zusätzlich als Antigen eine Peptidsequenz des Tetanustoxins (hierin als TTp bezeichnet). TTp stellt ein beispielhaftes antigen-haltiges Peptid dar und wurde durch die Klonierung eines von Tetanus-Toxin (TT) abgeleiteten antigenen Peptids (hierin TTp), welches die AA506-526 (gemäß SEQ ID No. 55) von TT umfasst, hergestellt. Dieses Konstrukt wird hierin auch mit [E7B6-TTp-E7B6-GFP-E7B6] (Aminosäuresequenz gemäß SEQ ID No. 35, Nukleinsäuresequenz gemäß SEQ ID No. 34) abgekürzt und enthalten ebenfalls das Peptid E7B6 in dreifacher Ausführung.

Die Klonierung der Linkerpeptide wurde wie folgt durchgeführt: Als Template für die PCR diente ein Vektor, der rekombinant die Aminsosäuresequenz der Aminosäuren 311-328 des humanen La Proteins (dies ist auch die Sequenz des Peptids E7B6 gemäß SEQ ID No. 23) enthielt. Durch Klonierung wurde ein neuer Klon mit zwei hintereinander liegenden E7B6-Sequenzen erzeugt, die durch eine künstliche AatII Restriktionsschnittstelle getrennt sind. In diese Schnittstelle wurde die Sequenz des EGFP-Leserahmens insertiert. Anschließend wurde die Nukleinsäuresequenz des Peptids E7B6 in diesen Vektor kloniert, wodurch das Konstrukt mit drei E7B6 Peptiden und einem GFP ([E7B6-E7B6-GFP-E7B6] mit Aminosäuresequenz gemäß SEQ ID No. 37) entstand.

Zur Herstellung des Konstrukts mit TTp wurde das TTp Peptid, zwischen das erste und zweite E7B6-Element des Vektors für [E7B6-E7B6-GFP-E7B6] insertiert. Die Moleküle wurden wie in Ausführungsbeispiel 2 beschrieben rekombinant exprimiert und aufgereinigt (Fig. 3).

Die in Ausführungsbeispiel 2 und 3 bereitgestellten Konstrukte wurden miteinander kombiniert, so dass zwei erfindungsgemäße Zusammensetzungen gebildet wurden:
- [DD2-7B6] und [E7B6-E7B6-GFP-E7B6], sowie
- [DD2-7B6] und [E7B6-TTp-E7B6-GFP-E7B6].

Der Antikörper 7B6 bindet spezifisch an das Peptid 7B6, so dass beide Fusionsproteine, die in der jeweiligen erfindungsgemäßen Zusammensetzung vorliegen, komplexieren.

### Ausführungsbeispiel 4: Bindung von Nukleinsäure an E7B6

Das Peptid E7B6, welches im Fusionsprotein aus Ausführungsbeispiel 3 enthalten ist, bindet an Nukleinsäuren. Zum Nachweis wurde die Nukleinsäurebindung mittels Oberflächen Plasmon Resonanz (SPR) bestimmt (Fig. 7 A-E). Dazu wurden verschiedene Nukleinsäuren (dsDNA, ssDNA, dsRNA, ssRNA) an einen handelsüblichen SPR-CHIP gekoppelt und mit Fusionsproteinen, die ein Peptid E7B6 enthalten, und Fusionsproteinen Peptid E7B6 (Vergleichsbeispiel) kontaktiert. Fusionsproteine mit E7B6 binden mit hoher Affinität an Nukleinsäuren vom Typ dsDNA (Fig. 7 A), ssDNA (Fig. 7 D), dsRNA (Fig. 7 B) und mit geringerer Affinität ssRNA (Fig. 7 C). Fusionsproteine ohne E7B6 (Fig. 6F) binden nicht an Nukleinsäuren. Die Bindung an Nukleinsäuren behindert nicht die Bindung von spezifischen Antikörpern an das Linkerpeptid. Fig. 7E zeigt die Bindung des 7B6-Antikörpers (monoklonaler IgG1), der spezifisch an das Peptid E7B6 bindet, nach Bindung an Nukleinsäuren. Die kürzeste gebundene Nukleinsäure war ein 15 mer ssDNA.

### Ausführungsbeispiel 5: Bindung einer erfindungsgemäßen Zusammensetzung mit [DD2-7B6] und [E7B6-E7B6-GFP-E7B6] an native slan-DC

Im Experiment wurden humane PBMC eingesetzt und nach Färbung mit fluoreszenzmarkierten Antikörper mittels FACS-Analyse analysiert. PBMC enthalten slan-DC, so dass die Bindung eines Komplexes aus [DD2-7B6] und [E7B6-E7B6-GFP-E7B6] an slan-DC getestet wurde. Als Vergleichsbeispiel wurden die slan-DC mit monoklonalem anti-slan IgM DD2 (Fig. 5a) und dem [E7B6-E7B6-GFP-E7B6] ohne [DD2-7B6] (Fig. 5b) angefärbt. Es ist ersichtlich, dass [E7B6-E7B6-GFP-E7B6] allein nicht an slan-DC bindet. Erfindungsgemäße Zusammensetzungen aus [DD2-7B6] und [E7B6-E7B6-GFP-E7B6] binden an slan-DC (Fig. 5c). Es erfolgte jeweils ein Costaining der untersuchten Proteine mit anti-CD 16.

### Ausführungsbeispiel 6: Bindung einer erfindungsgemäßen Zusammensetzung mit [DD2-7B6] und [E7B6-TTp-E7B6-GFP-E7B6] slan⁺ Jurkat Zellen

Im Experiment wurde als *in vitro* Modell für slan-positive Zellen eine Jurkat-Zellinie (hierin "slan⁺ Jurkat") verwendet, die das slan-Epitop auf der Oberfläche exprimiert (Schäkel et al., Immunity, 2002, 17, 289-301). Die Bindung der erfindungsgemäßen Zusammensetzung aus [DD2-7B6] und [E7B6-TTp-E7B6-GFP-E7B6], welche in der Zusammensetzung als Komplex vorliegen, wurde per FACS-Analyse ermittelt.

Als Vergleichsbeispiel wurden die slan⁺ Jurkat mit monoklonalem anti-slan IgM DD2 (Fig. 6a) und dem [E7B6-TTp-E7B6-GFP-E7B6] ohne [DD2-7B6] (Fig. 6b) angefärbt. Es ist ersichtlich, dass [E7B6-TTp-E7B6-GFP-E7B6] allein nicht an slan⁺ Jurkat bindet. In Fig. 6c ist die FACS-Färbung von slan⁺ Jurkat mit einem Komplex aus [DD2-7B6] und [E7B6-TTp-E7B6-GFP-E7B6] abgebildet. Die Bindung des Komplexes an die slan⁺ Jurkat war erfolgreich und nahezu so effektiv, wie die Bindung des als Vergleichsbeispiel eingesetzten monoklonalen anti-slan IgM DD2 Antikörpers.

### Ausführungsbeispiel 7: Vergleich der Bindung der scFv-Fragmente von DD2, MB4 und MB6 an slan-DC

Es wurden scFv-Fragmente der erfindungsgemäßen anti-slan Antikörper MB4 und MB6 (gemäß Ausführungsbeispiel 1) und ein scFv-Fragment des anti-slan Antikörpers DD2 (wie im bispezifischen Antikörper gemäß Ausführungsbeispiel 2 enthalten) bereitgestellt.

Per FACS-Analyse wurden die scFv-Fragmente auf ihre Bindung an das slan-Epitop getestet. Die Detektion der scFv erfolgte mit anti-c-myc-FITC (für MB4 und MB6) und mit anti-His Alexa488® für die scFv von DD2.

Die FACS-Analyse (Fig. 1B) zeigt, dass sowohl die monoklonalen anti-slan Antikörper MB6 und MB4 als auch die aus deren variablen Domänen der schweren und leichten Ketten konstruierten scFvs sehr gut an slan-DCs binden.

Die scFv des anti-slan Antikörpers DD2 binden in monovalenter Form schlecht an das slan-Epitop (Fig. 8c). Die Bindungsfähigkeit der DD2 Antikörper wird durch die Bereitstellung der scFv-Fragmente in einer erfindungsgemäßen Zusammensetzung, beispielsweise gemäß der Ausführungsbeispiele 5 und 6, rekonstituiert (Fig. 8b, in Form einer erfindungsgemäßen Zusammensetzung mit [DD2-7B6] und [E7B6-E7B6-GFP-E7B6] gemäß Ausführungsbeispiele 2 und 3). Die zeigt, dass durch die Bereitstellung in einer erfindungsgemäßen Zusammensetzung auch Antikörperfragmente, insbesondere scFv, mit einer niedrigen Affinität zum spezifischen Antigen geeignet sind. Die Vorteile der pentameren IgM Antikörper lassen sich somit in die erfindungsgemäße Zusammensetzung übertragen, wogegen die Nachteile der sperrigen IgM Antikörper, insbesondere die schwere Handhabbarkeit aufgrund deren Größe, überwunden werden.

### Ausführungsbeispiel 8: Aktivierung von slan-DC nach gezieltem Transport eines Co-Stimulus (TLR-Liganden) mit Hilfe einer erfindungsgemäßen Zusammensetzung mit [DD2-7B6] und [E7B6- E7B6-GFP-E7B6]

Für den Transport von Nukleinsäure aus *Escherichia coli* (einem aktivierenden Co-Stimulus, der an verschiedene Toll like Rezeptoren bindet) an slan-DC in einem *in vitro* Versuch wurde eine erfindungsgemäße Zusammensetzung mit
- dem anti-slan Antikörper: [DD2-7B6] (Aminosäuresequenz gemäß SEQ ID No. 33, Nukleinsäuresequenz gemäß SEQ ID No. 32) und
- dem drei E7B6-Bindungseinheiten enthaltenen Oligopeptid: [E7B6-E7B6-GFP-E7B6] (Aminosäuresequenz gemäß SEQ ID No. 37, Nukleinsäuresequenz gemäß SEQ ID No. 36)
eingesetzt. Die Aktivierung der slan-DC wurde durch die Bestimmung der TNF-α Konzentration im Zellkulturüberstand ermittelt. Diese wurde mit ELISA bestimmt.

### Es wurde wie folgt vorgegangen:

Nukleinsäuren wurden aus Totalextrakten von *E. coli* isoliert. Zur Beladung des die Nukleinsäure bindenden Bindungseinheit "E7B6" enthaltenden Oligopeptids [E7B6-E7B6-GFP-E7B6] wurde jeweils 1 mg des Oligopeptids [E7B6-E7B6-GFP-E7B6] mit jeweils 0,1 mg des aus *E. coli* gewonnenen Nukleinsäurepräparates für 1 h bei Raumtemperatur inkubiert. Dadurch ist ein Komplex erhältlich, in dem Nukleinsäure an [E7B6-E7B6-GFP-E7B6] gebunden ist (hierin NA-[E7B6-E7B6-GFP-E7B6]). Als Kontrolle wurde eine Probe des mit Nukleinsäure beladenen Oligopeptids NA-[E7B6-E7B6-GFP-E7B6] mit einer Mischung von RNAseA, T1 und DNAseI behandelt (Inkubation über Nacht). Dadurch wird die an das Oligopeptid gebundene Nukleinsäure verdaut, so dass nur das Oligopeptid verbleiben sollte. Die mit Nukleasen verdaute Probe wird fortan als NA*-[E7B6-E7B6-GFP-E7B6] bezeichnet. Anschließend wurden beide Proben NA-[E7B6-E7B6-GFP-E7B6] und NA*-[E7B6-E7B6-GFP-E7B6] über eine Nickel-Affinitätssäule erneut gereinigt, wodurch verdaute und ungebundete Nukleinsäurebestandteile vom Oligopeptid abgetrennt wurden.

Anschließend wurden die beiden Proben NA-[E7B6-E7B6-GFP-E7B6] und NA*-[E7B6-E7B6-GFP-E7B6] in separaten Ansätzen jeweils im molaren Verhältnis 1:3 (Oligopeptid zu bispezifischen Antikörpern) mit den bispezifischen anti-slan Antikörper [DD2-7B6] versetzt und für 2h bei Raumtemperatur inkubiert. Dabei erfolgt eine Komplexbildung zwischen dem jeweiligen Oligopeptid mit der E7B6-Domäne und dem bispezifischen Antikörper [DD2-7B6], der mit dem 7B6 Teil an das E7B6-Peptid bindet. Die dadurch erhältlichen Komplexe werden hierin vereinfacht wie folgt bezeichnet:
- Komplex NA-[E7B6-E7B6-GFP-E7B6] und [DD2-7B6]: NA-DD2
- Komplex NA*-[E7B6-E7B6-GFP-E7B6] und [DD2-7B6]: NA*-DD2

Aliquots der Komplexe NA-DD2 (erfindungsgemäß) und NA*-DD2 (Negativkontrolle) (enthaltend jeweils 2,8 nMol des bispezifischen Antikörpers [DD2-7B6]) wurden im *in vitro* Versuch mit frisch isolierten slan-DC (jeweils 1,4x10⁶ DCs) versetzt und über Nacht inkubiert. Als weitere Kontrollen wurde unter identischen Bedingungen ein Komplex aus [E7B6-E7B6-GFP-E7B6] (ohne Nukleinsäurebeladung, ohne Verdau) mit [DD2-7B6] eingesetzt (Negativkontrolle, hierin wird der Komplex als *-DD2 bezeichnet). Weiter wurden slan-DC mit dem TLR4 Liganden LPS (ohne Komplex) unter ansonsten identischen Bedingungen stimuliert, was eine Aktivierung der slan-DC zur Folge hat (Positivkontrolle). Der Zellkulturüberstand wurde anschließend abgenommen und mittels TNF-α analysiert.

Fig. 9 zeigt die Ergebnisse des anti-TNFα ELISAs. Die erfindungsgemäße Zusammensetzung ist zum Transport von Co-Stimuli, wie die hier getesteten Toll like Rezeptor Liganden (Nukleinsäuren), an slan-DC geeignet, wodurch eine Aktivierung der slan-DC bewirkt wird.

## Patentansprüche

1. Anti-slan Antikörper, welcher spezifisch an humane dendritische Zellen bindet, deren Oberfläche den P Selectin Glycoprotein Ligand 1 (PSGL-1) mit der O-gebundenen Glykanstruktur 6-sulfo N-Acetly-Lactosamin (6-sulfo LacNAc) aufweist, wobei der Antikörper komplementaritätsbestimmende Regionen (complementarity determining regions, CDRs) aufweist, wobei die CDRs die folgenden Aminosäuresequenzen umfassen:
a) variable Region der schweren Kette (V_{H}):
CDR1 TYGVH (SEQ ID No. 1),
CDR2 VIWSGGGTDFNVAFXS (SEQ ID No. 2),
wobei X ausgewählt ist aus M, L, F, oder I, vorzugsweise aus M oder I,
CDR3 RTTNDGNYAFAY (SEQ ID No. 5), und
b) variable Region der leichten Kette (V_{L}):
CDR1 RSSQNILHSDGXTYLE (SEQ ID No. 6),
wobei X ausgewählt ist aus S, T, N, Q, H, K oder R, vorzugsweise aus S oder N,
CDR2 KVSNRFS (SEQ ID No. 9) und
CDR3 FQGSHVPWT (SEQ ID No. 10).
wobei der Antikörper folgende Struktur umfasst:
- variable Region der schweren Kette gemäß SEQ ID No. 12 und variable Region der leichten Kette gemäß SEQ ID No. 14 oder
- variable Region der schweren Kette gemäß SEQ ID No. 16 und variable Region der leichten Kette gemäß SEQ ID No. 18
oder eine entsprechende humanisierte Struktur.

2. Zusammensetzung, umfassend
a) mindestens einen anti-slan Antikörper nach Anspruch 1,
b) einen Co-Stimulus, der an einen für den Co-Stimulus spezifischen Pattern Recognition Rezeptor auf dendritischen Zellen bindet und dadurch die Aktivierung, von besagten dendritischen Zellen bewirkt, oder mindestens eine Bindungseinheit, die an diesen Co-Stimulus spezifisch bindet,
c) mindestens ein Antigen.

3. Zusammensetzung nach Anspruch 2 zusätzlich enthaltend
d) mindestens einen weiteren Antikörper, der spezifisch mindestens eines der Bestandteile a), b) oder c) der Zusammensetzung bindet, als weitere Bindungseinheit.

4. Zusammensetzung nach einem der vorhergehenden Ansprüche, **dadurch gekennzeichnet, dass** die Bindungseinheit und/oder die weitere Bindungseinheit mit dem anti-slan Antikörper und/oder mit dem Antigen über eine kovalente Bindung, bevorzugt eine Peptidbindung, verbunden ist.

5. Zusammensetzung nach einem der Ansprüche 2 bis 4 , **dadurch gekennzeichnet, dass**
- der anti-slan Antikörper und die Bindungseinheit als Fusionsprotein vorliegen oder das Antigen und die Bindungseinheit als Fusionsprotein vorliegen und/oder
- der anti-slan Antikörper und die Bindungseinheit als Fusionsprotein vorliegen und die weitere Bindungseinheit und das Antigen als Fusionsprotein vorliegen oder
- der anti-slan Antikörper und die weitere Bindungseinheit als Fusionsprotein vorliegen und das Antigen und die Bindungseinheit als Fusionsprotein vorliegen.

6. Zusammensetzung nach einem der Ansprüche 2 bis 5, **dadurch gekennzeichnet, dass** die Bindungseinheit ein Peptid ist, welches bevorzugt die folgende Aminosäuresequenz aufweist: EKEALKKIIEDQQESLNK (SEQ ID No. 23), und die weitere Bindungseinheit ein Antikörper ist, der das Peptid spezifisch bindet.

7. Zusammensetzung nach Anspruch 6, **dadurch gekennzeichnet, dass** der Antikörper, der das Peptid spezifisch bindet, die folgenden CDR-Regionen umfasst:
- variable Region der schweren Kette (V_{H}): CDR1 SEQ ID No. 26, CDR2 SEQ ID No. 27, CDR3 SEQ ID No. 28 und
- variable Region der leichten Kette (V_{L}): CDR1 SEQ ID No. 29, CDR2 SEQ ID No. 30, CDR3 SEQ ID No. 31.

8. Zusammensetzung nach einem der Ansprüche 2 bis 7, **dadurch gekennzeichnet, dass** das Antigen ein Tumorantigen ist.

9. Nukleinsäuresequenz kodierend für einen anti-slan Antikörper nach Anspruch 1.

10. Expressionsvektor enthaltend eine Nukleinsäuresequenz nach Anspruch 9.

11. Wirtszelle oder nicht-menschlicher Wirtsorganismus, rekombinant enthaltend eine Nukleinsäuresequenz nach Anspruch 9 oder einen Expressionsvektor nach Anspruch 10.

12. Pharmazeutischer Kit, enthaltend eine Zusammensetzung nach einem der Ansprüche 2 bis 8 und einen Liganden, der die Aktivierung von humanen dendritischen Zellen, bewirkt in Assoziation mit einem pharmazeutisch annehmbaren Verdünnungsmittel oder Träger, gegebenenfalls in einer für die intravenöse Verabreichung geeigneten Form.

13. Pharmazeutischer Kit nach Anspruch 12 zur Verwendung in der therapeutischen Behandlung von Tumorerkrankungen, wobei das Antigen ein Tumorantigen ist.

## Claims

1. Anti-slan antibody, which binds specifically to human dendritic cells whose surface has the P-selectin glycoprotein ligand 1 (PSGL-1) with O-bound glycan structure 6-sulfo N-acetyl lactosamine (6-sulfo LacNAc), the antibody comprising (CDRs), wherein the CDRs comprise the following amino acid sequences:
a) variable region of the heavy chain (V_{H}):
CDR1 TYGVH (SEQ ID No. 1),
CDR2 VIWSGGGTDFNVAFXS (SEQ ID No. 2),
wherein X is selected from M, L, F, or I, preferably from M or I,
CDR3 RTTNDGNYAFAY (SEQ ID No. 5), and
b) variable region of the light chain (V_{L}):
CDR1 RSSQNILHSDGXTYLE (SEQ ID No. 6),
wherein X is selected from S, T, N, Q, H, K or R, preferably from S or N,
CDR2 KVSNRFS (SEQ ID No. 9) and
CDR3 FQGSHVPWT (SEQ ID No. 10).
whereas the antibody comprises the following structure:
- variable region of the heavy chain according to SEQ ID No. 12 and variable region of the light chain according to SEQ ID No. 14, or
- variable region of the heavy chain according to SEQ ID No. 16 and variable region of the light chain according to SEQ ID No. 18.
or a corresponding humanized structure.

2. Composition comprising
a) at least one anti-slan antibody according to claim 1,
b) a co-stimulus which binds to a receptor on dendritic cells and thereby causes the activation of said dendritic cells, or at least one binding unit, that binds at least one binding unit that binds specifically to the co-stimulus,
c) at least one antigen.

3. Composition according to claim 2, comprising additionally
d) at least one further antibody, which binds specifically at least one of the components a), b), or c) of the composition, as further binding unit.

4. Composition according to one of the preceding claims, **characterized in that** the binding unit and/or the further binding unit is bound to the anti-slan antibody and/or the antigen by a covalent bond, preferably a peptide bond.

5. Composition according to one of the claims 2 to 4, **characterized in that**
- the anti-slan antibody and the binding unit are present as a fusion protein or the antigen and the binding unit are present as a fusion protein, and/or
- the anti-slan antibody and the binding unit are present as a fusion protein and the further binding unit and the antigen are present as a fusion protein or
- the anti-slan antibody and the further binding unit are present as a fusion protein and the antigen and the binding unit are present as a fusion protein.

6. Composition according to one of the Claims 2 to 5, **characterized in that** the binding unit is a peptide which preferably has the following amino acid sequence: EKEALKKIIEDQQESLNK (SEQ ID No. 23) and the further binding unit is an antibody which binds the peptide specifically.

7. Composition according to claim 6, **characterized in that** the antibody, which binds the peptide specifically comprises the following CDR regions:
- variable region of the heavy chain (V_{H}): CDR1 SEQ ID No. 26, CDR2 SEQ ID No. 27, CDR3 SEQ ID No. 28, and
- variable region of the light chain (V_{L}): CDR1 SEQ ID No. 29, CDR2 SEQ ID No. 30, CDR3 SEQ ID No. 31.

8. Composition according to one of the claims 1 to 7, **characterized in that** the antigen is a tumor antigen.

9. Nucleic acid sequence coding for an anti-slan antibody as defined in claim 1.

10. Expression vector containing a nucleic acid sequence according to claim 9.

11. Host cell or non-human host organism, recombinantly containing a nucleic acid sequence according to claim 9 or an expression vector according to claim 10.

12. Pharmaceutical kit, containing a composition according to one of the claims 2 to 8 and a ligand that effects the activation of human dendritic cells in association with a pharmaceutically suitable diluent or carrier, optionally in a form suitable for intravenous administration.

13. Pharmaceutical kit according to claim 12 for use in the therapeutic treatment of tumor illnesses, wherein the antigen is a tumor antigen.

## Revendications

1. Anticorps anti-slan, qui se lie spécifiquement à des cellules dendritiques humaines, dont la surface présente le ligand glycoprotéique 1 de la P-sélectine (PSGL-1) avec la 6 sulfo N-acétyl lactosamine à structure glycannique liée par O (6-sulfo LacNAc), l'anticorps comprenant des régions déterminant la complémentarité (complementarity determining regions, CDR), les CDR comprenant les séquences d'acides aminés suivantes :
a) région variable de la chaîne lourde (V_{H}) :
CDR1 TYGVH (SEQ ID N°1),
CDR2 VIWSGGGTDFNVAFXS (SEQ ID N°2),
où X est choisi parmi M, L, F ou I, de préférence parmi M ou I,
CDR3 RTTNDGNYAFAY (SEQ ID N°5), et
b) région variable de la chaîne légère (V_{L}) :
CDR1 RSSQNILHSDGXTYLE (SEQ ID N°6)
où X est choisi parmi S, T, N, Q, H, K ou R, de préférence parmi S ou N,
CDR2 KVSNRFS (SEQ ID N°9) et
CDR3 FQGSHVPWT (SEQ ID N°10),
l'anticorps comprenant la structure suivante :
- région variable de la chaîne lourde selon SEQ ID N°12 et région variable de la chaîne légère selon SEQ ID N°14 ou
- région variable de la chaîne lourde selon SEQ ID N°16 et région variable de la chaîne légère selon SEQ ID N°18
ou une structure humanisée correspondante.

2. Composition, comprenant
a) au moins un anticorps anti-slan selon la revendication 1,
b) un co-stimulus, qui se lie à un récepteur de reconnaissance de motifs spécifique au co-stimulus sur des cellules dendritiques et provoque ainsi l'activation desdites cellules dendritiques, ou au moins une unité de liaison, qui se lie spécifiquement à ce co-stimulus,
c) au moins un antigène.

3. Composition selon la revendication 2, contenant en plus
d) au moins un autre anticorps, qui se lie spécifiquement au moins à l'un des composants a), b) ou c) de la composition, comme unité de liaison supplémentaire.

4. Composition selon l'une des revendications précédentes, **caractérisée en ce que** l'unité de liaison et/ou l'unité de liaison supplémentaire sont liées à l'anticorps anti-slan et/ou à l'antigène au moyen d'une liaison covalente, de préférence une liaison peptidique.

5. Composition selon l'une des revendications 2 à 4, **caractérisée en ce que**
- l'anticorps anti-slan et l'unité de liaison se présentent sous forme de protéine de fusion ou l'antigène et l'unité de liaison se présentent sous forme de protéine de fusion et/ou
- l'anticorps anti-slan et l'unité de liaison se présentent sous forme de protéine de fusion et l'unité de liaison supplémentaire et l'antigène se présentent sous forme de protéine de fusion ou
- l'anticorps anti-slan et l'unité de liaison supplémentaire se présentent sous forme de protéine de fusion et l'antigène et l'unité de liaison se présentent sous forme de protéine de fusion.

6. Composition selon l'une des revendications 2 à 5, **caractérisée en ce que** l'unité de liaison est un peptide, lequel présente de préférence la séquence d'acides aminés suivante : EKEALKKIIEDQQESLNK (SEQ ID N°23), et l'unité de liaison supplémentaire est un anticorps, qui se lie spécifiquement au peptide.

7. Composition selon la revendication 6, **caractérisée en ce que** l'anticorps, qui se lie spécifiquement au peptide, comprend les régions CDR suivantes :
- région variable de la chaîne lourde (V_{H}) : CDR1 SEQ ID N°26, CDR2 SEQ ID N°27, CDR3 SEQ ID N°28 et
- région variable de la chaîne légère (V_{L}) : CDR1 SEQ ID N°29, CDR2 SEQ ID N°30, CDR3 SEQ ID N°31.

8. Composition selon l'une des revendications 2 à 7, **caractérisée en ce que** l'antigène est un antigène tumoral.

9. Séquence d'acides nucléiques codant pour un anticorps anti-slan selon la revendication 1.

10. Vecteur d'expression contenant une séquence d'acides nucléiques selon la revendication 9.

11. Cellule hôte ou organisme hôte non humain, contenant de manière recombinante une séquence d'acides nucléiques selon la revendication 9 ou un vecteur d'expression selon la revendication 10.

12. Kit pharmaceutique, contenant une composition selon l'une des revendications 2 à 8 et un ligand, qui provoque l'activation de cellules dendritiques humaines, en association avec un diluant ou un support pharmaceutiquement acceptable, le cas échéant sous une forme appropriée pour l'administration intraveineuse.

13. Kit pharmaceutique selon la revendication 12 destiné à être utilisé dans le traitement thérapeutique d'affections tumorales, l'antigène étant un antigène tumoral.


### SEQUENCE LISTING

<110> Technische Universität Dresden
<120> Antikörper gegen 6-sulfo LacNAc positive humane dendritische Zellen und deren Verwendung
<130> 00017P0141DEWO
<150> DE 10 2010 039 019.4-41
   <151> 2010-08-06
<160> 55
<170> PatentIn version 3.5
<210> 1
   <211> 5
   <212> PRT
   <213> Mus musculus
<400> 1
   Thr Tyr Gly Val His
   1               5
<210> 2
   <211> 16
   <212> PRT
   <213> Mus musculus
<220>
   <221> misc_feature
   <222> (15)..(15)
   <223> Xaa can be any naturally occurring amino acid
<400> 2
   Val Ile Trp Ser Gly Gly Gly Thr Asp Phe Asn Val Ala Phe Xaa Ser
   1 5 10 15
<210> 3
   <211> 16
   <212> PRT
   <213> Mus musculus
<400> 3
   Val Ile Trp Ser Gly Gly Gly Thr Asp Phe Asn Val Ala Phe Met Ser
   1 5 10 15
<210> 4
   <211> 16
   <212> PRT
   <213> Mus musculus
<400> 4
   Val Ile Trp Ser Gly Gly Gly Thr Asp Phe Asn Val Ala Phe Ile Ser
   1 5 10 15
<210> 5
   <211> 12
   <212> PRT
   <213> Mus musculus
<400> 5
   Arg Thr Thr Asn Asp Gly Asn Tyr Ala Phe Ala Tyr
   1 5 10
<210> 6
   <211> 16
   <212> PRT
   <213> Mus musculus
<220>
   <221> misc_feature
   <222> (12)..(12)
   <223> Xaa can be any naturally occurring amino acid
<400> 6
   Arg Ser Ser Gln Asn Ile Leu His Ser Asp Gly Xaa Thr Tyr Leu Glu
   1 5 10 15
<210> 7
   <211> 16
   <212> PRT
   <213> Mus musculus
<400> 7
   Arg Ser Ser Gln Asn Ile Leu His Ser Asp Gly Ser Thr Tyr Leu Glu
   1 5 10 15
<210> 8
   <211> 16
   <212> PRT
   <213> Mus musculus
<400> 8
   Arg Ser Ser Gln Asn Ile Leu His Ser Asp Gly Asn Thr Tyr Leu Glu
   1 5 10 15
<210> 9
   <211> 7
   <212> PRT
   <213> Mus musculus
<400> 9
   Lys Val Ser Asn Arg Phe Ser
   1 5
<210> 10
   <211> 9
   <212> PRT
   <213> Mus musculus
<400> 10
   Phe Gln Gly Ser His Val Pro Trp Thr
   1 5
<210> 11
   <211> 357
   <212> DNA
   <213> Mus musculus
<400> 11
   gaggtcaagc tgcaggagtc aggacctggc ctagtgcagc cctcacagag cctgtccatc 60
   acctgcacag tctctggttt ctcattaact acctatggtg tacactggat tcgccagtct 120
   ccaggaaagg gtctggagtg gctgggagtg atatggagtg gtggaggtac tgactttaat 180
   gtagctttca tgtccagact gagcatcaac aaggacaatt ccaagagcca aattttcttt 240
   aaaatgaaca gtctgcaagc taatgacaca gccatatatt attgtgccag aagaacgacc 300
   aacgatggta actacgcttt tgcttattgg ggccaaggga ctctggtcac tgtctct 357
<210> 12
   <211> 119
   <212> PRT
   <213> Mus musculus
<400> 12
   Glu Val Lys Leu Gln Glu Ser Gly Pro Gly Leu Val Gln Pro Ser Gln
   1               5                   10                  15
   Ser Leu Ser Ile Thr Cys Thr Val Ser Gly Phe Ser Leu Thr Thr Tyr
               20                  25                  30
   Gly Val His Trp Ile Arg Gln Ser Pro Gly Lys Gly Leu Glu Trp Leu
           35                  40                  45
   Gly Val Ile Trp Ser Gly Gly Gly Thr Asp Phe Asn Val Ala Phe Met
       50                  55                  60
   Ser Arg Leu Ser Ile Asn Lys Asp Asn Ser Lys Ser Gln Ile Phe Phe
   65                  70                  75                  80
   Lys Met Asn Ser Leu Gln Ala Asn Asp Thr Ala Ile Tyr Tyr Cys Ala
                   85                  90                  95
   Arg Arg Thr Thr Asn Asp Gly Asn Tyr Ala Phe Ala Tyr Trp Gly Gln
               100                 105                 110
   Gly Thr Leu Val Thr Val Ser
           115
<210> 13
   <211> 363
   <212> DNA
   <213> Mus musculus
<400> 13
   gatattgtga tgacacagtc tccactctcc ctgcctgtca gtcttggaga tcaagcctcc 60
   atctcttgca gatctagtca gaacatttta catagtgatg gaagcaccta tttagaatgg 120
   tacctgcaga aaccaggcca gtctccaaag ctcctgatct acaaagtttc caaccgattt 180
   tctggggtcc cagacaggtt cagtggcagt ggatcaggga cagatttcac actcaagatc 240
   agcagagtgg aggctgagga tctgggagtt tattactgct ttcagggttc acatgttccg 300
   tggacgttcg gtggaggcac caagctggaa atcaaacggg ctgatgctgc accaactgta 360
   tcc 363
<210> 14
   <211> 121
   <212> PRT
   <213> Mus musculus
<400> 14
   Asp Ile Val Met Thr Gln Ser Pro Leu Ser Leu Pro Val Ser Leu Gly
   1               5                   10                  15
   Asp Gln Ala Ser Ile Ser Cys Arg Ser Ser Gln Asn Ile Leu His Ser
               20                  25                  30
   Asp Gly Ser Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Val Pro Trp Thr Phe Gly Gly Gly Thr Lys Leu Glu Ile Lys
               100                 105                 110
   Arg Ala Asp Ala Ala Pro Thr Val Ser
           115                 120
<210> 15
   <211> 357
   <212> DNA
   <213> Mus musculus
<400> 15
   gaagtcaagc tggagcagtc aggacctggc ctagtgcagc tctcacagag cctgtccatc 60
   acctgcacag tctctggttt ctcattaact acctatggtg tacactggat tcgccagtct 120
   ccaggaaagg gtctggagtg gctgggagtg atatggagtg gtggaggtac tgactttaat 180
   gtagctttca tatccagact gagcatcaac aaggacaatt ccaagagcca agttttcttt 240
   aaaatgaaca gtctgcaagc taatgacaca gccatatatt actgtgccag aagaacgacc 300
   aacgatggta actacgcttt tgcttactgg ggccaaggga ctctggtcac tgtctct 357
<210> 16
   <211> 119
   <212> PRT
   <213> Mus musculus
<400> 16
   Glu Val Lys Leu Glu Gln Ser Gly Pro Gly Leu Val Gln Leu Ser Gln
   1               5                   10                  15
   Ser Leu Ser Ile Thr Cys Thr Val Ser Gly Phe Ser Leu Thr Thr Tyr
               20                  25                  30
   Gly Val His Trp Ile Arg Gln Ser Pro Gly Lys Gly Leu Glu Trp Leu
           35                  40                  45
   Gly Val Ile Trp Ser Gly Gly Gly Thr Asp Phe Asn Val Ala Phe Ile
       50                  55                  60
   Ser Arg Leu Ser Ile Asn Lys Asp Asn Ser Lys Ser Gln Val Phe Phe
   65                  70                  75                  80
   Lys Met Asn Ser Leu Gln Ala Asn Asp Thr Ala Ile Tyr Tyr Cys Ala
                   85                  90                  95
   Arg Arg Thr Thr Asn Asp Gly Asn Tyr Ala Phe Ala Tyr Trp Gly Gln
               100                 105                 110
   Gly Thr Leu Val Thr Val Ser
           115
<210> 17
   <211> 363
   <212> DNA
   <213> Mus musculus
<400> 17
   gatattgtga tgacacagtc tccactctcc ctgcctgtca gtcttggaga tcaagcctcc      60
   atctcttgca gatctagtca gaacatttta catagtgatg gaaacaccta tttagaatgg     120
   tacctgcaga aaccaggcca gtctccaaag ctcctgatct acaaagtttc caaccgattt     180
   tctggggtcc cagacaggtt cagtggcagt ggatcaggga cagatttcac actcaagatc     240
   agcagagtgg aggctgagga tctgggagtt tattactgct ttcaaggttc acatgttccg     300
   tggacgttcg gtggaggcac caagctggaa atcaaacggg ctgatgctgc accaactgta     360
   tcc                                                                   363
<210> 18
   <211> 121
   <212> PRT
   <213> Mus musculus
<400> 18
   Asp Ile Val Met Thr Gln Ser Pro Leu Ser Leu Pro Val Ser Leu Gly
   1               5                   10                  15
   Asp Gln Ala Ser Ile Ser Cys Arg Ser Ser Gln Asn Ile Leu His Ser
               20                  25                  30
   Asp Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Val Pro Trp Thr Phe Gly Gly Gly Thr Lys Leu Glu Ile Lys
               100                 105                 110
   Arg Ala Asp Ala Ala Pro Thr Val Ser
           115                 120
<210> 19
   <211> 130
   <212> PRT
   <213> Mus musculus
<400> 19
   Met Ala Asp Tyr Lys Gln Val Lys Leu Gln Gln Ser Gly Ala Glu Leu
   1               5                   10                  15
   Val Lys Pro Gly Ala Ser Val Lys Leu Ser Cys Lys Ala Ser Gly Tyr
               20                  25                  30
   Thr Phe Thr Thr Tyr Tyr Met Tyr Trp Val Lys Gln Arg Pro Gly Gln
   35 40 45
   Gly Leu Glu Trp Ile Gly Glu Ile Ser Pro Ile Asn Gly Ala Thr Asn
   50 55 60
   Phe Asn Glu Lys Phe Lys Ser Lys Ala Thr Leu Thr Val Asp Lys Ser
   65 70 75 80
   Ser Ser Thr Ala Tyr Met Gln Leu Ser Ser Leu Thr Ser Glu Asp Ser
   85 90 95
   Ala Val Tyr Tyr Cys Thr Thr Leu Gly Glu Lys Gly Phe Ala Tyr Trp
   100 105 110
   Gly Gln Gly Thr Leu Val Thr Val Ser Ala Glu Ser Gln Ser Phe Pro
   115 120 125
   Asn Val
   130
<210> 20
   <211> 121
   <212> PRT
   <213> Mus musculus
<400> 20
   Asp Ile Val Leu Thr Gln Ser Pro Leu Ser Leu Pro Val Ser Leu Gly
   1               5                   10                  15
   Asp Gln Ala Ser Ile Ser Cys Arg Ser Ser Gln Ser Leu Val His Ser
               20                  25                  30
   Asn Gly Asn Thr Tyr Leu His Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Phe Cys Ser Gln Ser
                   85                  90                  95
   Thr His Val Pro Phe Thr Phe Gly Ser Gly Thr Lys Leu Glu Ile Lys
               100                 105                 110
   Arg Ala Asp Ala Ala Pro Thr Val Ser
           115                 120
<210> 21
   <211> 114
   <212> PRT
   <213> Mus musculus
<400> 21
   Gln Val Gln Leu Gln Gln Ser Gly Ala Glu Leu Val Lys Pro Gly Ala
   1               5                   10                  15
   Ser Val Lys Leu Ser Cys Lys Ala Ser Gly Tyr Thr Leu Thr Ser Tyr
               20                  25                  30
   Trp Leu His Trp Val Lys Gln Trp Pro Gly Arg Gly Leu Glu Trp Ile
           35                  40                  45
   Gly Arg Ile Asp Pro Asn Ser Gly Gly Thr Lys Tyr Asp Glu Lys Phe
       50                  55                  60
   Lys Ser Lys Ala Thr Leu Thr Val Asp Lys Pro Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Trp Asp Tyr Trp Gly Gly Ile Asn Gly Thr Thr Val Thr Val
               100                 105                 110
   Ser Ser
<210> 22
   <211> 110
   <212> PRT
   <213> Mus musculus
<400> 22
   Asp Ile Gln Leu Thr Gln Ser Pro Ala Ile Met Ser Ala Ser Pro Gly
   1               5                   10                  15
   Glu Lys Val Thr Met Thr Cys Arg Ala Ser Ser Ser Val Ser Ser Ser
               20                  25                  30
   Tyr Leu His Trp Tyr Gln Gln Lys Ser Gly Ala Ser Pro Lys Leu Trp
           35                  40                  45
   Ile Tyr Ser Thr Ser Asn Leu Ala Ser Gly Val Pro Ala Arg Phe Ser
       50                  55                  60
   Gly Ser Gly Ser Gly Thr Ser Tyr Ser Leu Thr Ile Ser Ser Val Glu
   65                  70                  75                  80
   Ala Glu Asp Ala Ala Thr Tyr Tyr Cys Gly Ile Asn Gln Tyr Ser Gly
                   85                  90                  95
   Tyr Pro Tyr Thr Phe Gly Gly Gly Thr Lys Leu Glu Ile Lys
               100                 105                 110
<210> 23
   <211> 18
   <212> PRT
   <213> Homo sapiens
<400> 23 Asn Lys
<210> 24
   <211> 42
   <212> PRT
   <213> Homo sapiens
<400> 24
   Trp Glu Val Leu Glu Gly Glu Val Glu Lys Glu Ala Leu Lys Lys Ile
   1               5                   10                  15
   Ile Glu Asp Gln Gln Glu Ser Leu Asn Lys Trp Lys Ser Lys Gly Arg
               20                  25                  30
   Arg Phe Lys Gly Lys Gly Lys Gly Asn Lys
           35                  40
<210> 25
   <211> 408
   <212> PRT
   <213> Homo sapiens
<400> 25
   Met Ala Glu Asn Gly Asp Asn Glu Lys Met Ala Ala Leu Glu Ala Lys
   1               5                   10                  15
   Ile Cys His Gln Ile Glu Tyr Tyr Phe Gly Asp Phe Asn Leu Pro Arg
               20                  25                  30
   Asp Lys Phe Leu Lys Glu Gln Ile Lys Leu Asp Glu Gly Trp Val Pro
           35                  40                  45
   Leu Glu Ile Met Ile Lys Phe Asn Arg Leu Asn Arg Leu Thr Thr Asp
       50                  55                  60
   Phe Asn Val Ile Val Glu Ala Leu Ser Lys Ser Lys Ala Glu Leu Met
   65                  70                  75                  80
   Glu Ile Ser Glu Asp Lys Thr Lys Ile Arg Arg Ser Pro Ser Lys Pro
                   85                  90                  95
   Leu Pro Glu Val Thr Asp Glu Tyr Lys Asn Asp Val Lys Asn Arg Ser
               100                 105                 110
   Val Tyr Ile Lys Gly Phe Pro Thr Asp Ala Thr Leu Asp Asp Ile Lys
           115                 120                 125
   Glu Trp Leu Glu Asp Lys Gly Gln Val Leu Asn Ile Gln Met Arg Arg
       130                 135                 140
   Thr Leu His Lys Ala Phe Lys Gly Ser Ile Phe Val Val Phe Asp Ser
   145                 150                 155                 160
   Ile Glu Ser Ala Lys Lys Phe Val Glu Thr Pro Gly Gln Lys Tyr Lys
                   165                 170                 175
   Glu Thr Asp Leu Leu Ile Leu Phe Lys Asp Asp Tyr Phe Ala Lys Lys
               180                 185                 190
   Asn Glu Glu Arg Lys Gln Asn Lys Val Glu Ala Lys Leu Arg Ala Lys
           195                 200                 205
   Gln Glu Gln Glu Ala Lys Gln Lys Leu Glu Glu Asp Ala Glu Met Lys
       210                 215                 220
   Ser Leu Glu Glu Lys Ile Gly Cys Leu Leu Lys Phe Ser Gly Asp Leu
   225                 230                 235                 240
   Asp Asp Gln Thr Cys Arg Glu Asp Leu His Ile Leu Phe Ser Asn His
                   245                 250                 255
   Gly Glu Ile Lys Trp Ile Asp Phe Val Arg Gly Ala Lys Glu Gly Ile
               260                 265                 270
   Ile Leu Phe Lys Glu Lys Ala Lys Glu Ala Leu Gly Lys Ala Lys Asp
           275                 280                 285
   Ala Asn Asn Gly Asn Leu Gln Leu Arg Asn Lys Glu Val Thr Trp Glu
       290                 295                 300
   Val Leu Glu Gly Glu Val Glu Lys Glu Ala Leu Lys Lys Ile Ile Glu
   305                 310                 315                 320
   Asp Gln Gln Glu Ser Leu Asn Lys Trp Lys Ser Lys Gly Arg Arg Phe
   325 330 335
   Lys Gly Lys Gly Lys Gly Asn Lys Ala Ala Gln Pro Gly Ser Gly Lys
   340 345 350
   Gly Lys Val Gln Phe Gln Gly Lys Lys Thr Lys Phe Ala Ser Asp Asp
   355 360 365
   Glu His Asp Glu His Asp Glu Asn Gly Ala Thr Gly Pro Val Lys Arg
   370 375 380
   Ala Arg Glu Glu Thr Asp Lys Glu Glu Pro Ala Ser Lys Gln Gln Lys
   385 390 395 400
   Thr Glu Asn Gly Ala Gly Asp Gln
   405
<210> 26
   <211> 5
   <212> PRT
   <213> Mus musculus
<400> 26
   Asp Phe Trp Met Asn
   1 5
<210> 27
   <211> 19
   <212> PRT
   <213> Mus musculus
<400> 27
   Gln Ile Arg Asn Lys Pro Asn Asn Tyr Glu Thr Tyr Tyr Ser Asp Ser
   1 5 10 15
   Leu Lys Gly
<210> 28
   <211> 8
   <212> PRT
   <213> Mus musculus
<400> 28
   Leu Gly Asn Ser Trp Phe Ala Tyr
   1 5
<210> 29
   <211> 17
   <212> PRT
   <213> Mus musculus
<400> 29
   Arg Ser Ser Gln Ser Leu Leu Asp Ser Arg Thr Arg Lys Asn Tyr Leu
   1 5 10 15
   Ala
<210> 30
   <211> 7
   <212> PRT
   <213> Mus musculus
<400> 30
   Trp Ala Ser Thr Arg Glu Ser
   1 5
<210> 31
   <211> 8
   <212> PRT
   <213> Mus musculus
<400> 31
   Lys Gln Ser Tyr Asn Leu Pro Thr
   1 5
<210> 32
   <211> 1758
   <212> DNA
   <213> Artificial Sequence
<220>
   <223> Fusionsprotein
<400> 32
   atggagacag acacactcct gctatgggta ctgctgctct gggttccagg ttccactggt      60
   gacgcggccc agccggccat ggcggactac aaacaggtta agctgcagca gtctggggct     120
   gaattggtga agcctggggc ttcagtgaag ttgtcctgca aggcttctgg ctacaccttc     180
   accacctact atatgtactg ggtgaagcag aggcctggac aaggccttga atggattgga     240
   gagattagtc ctataaatgg tgctactaac ttcaatgaga agttcaagag caaggccaca     300
   ctgactgtag acaaatcctc cagcacagca tacatgcaac tcagcagcct gacatctgag     360
   gactctgcgg tctattactg tacaacactg ggagagaagg gttttgctta ctggggccaa     420
   gggactctgg tcactgtctc tgcagagagt cagtccttcc caaatgtcgg tggtggtgga     480
   tccgacattg tgctcacaca gtctccatcc tccctggctg tgtcagcagg agagaaggtc     540
   actatgagct gcagatccag tcagagtctg ctcgacagta gaacccgaaa gaactacttg     600
   gcttggtacc agcagaaacc agggcagtct cctaaactgc tgatctactg ggcatccact     660
   agggaatctg gggtccctga tcgcttcaca ggcagtggat ctgggacaga tttcactctc     720
   accatcagca atgtgcaggc tgaagacctg gcagtttatt actgcaagca atcttataat     780
   cttccgacgt tcggtggagg caccaagctg gaaatcaaac gggctgatgc tgcaccaact     840
   gtatccgggc ccggtggtgg tggttctggt ggtggtggtt ctggcgccgg cggctccggt     900
   ggtggtggtt ccggtggtgg tggctccatg gcggactaca aagaggtcaa gctgcaggag     960
   tcaggaggag gcttggtgca acctgggagg cccatgaaac tctcctgtgt tgcctctgga    1020
   ttcactttta gtgacttctg gatgaactgg gtccgccagt ctccagagaa aggactggag    1080
   tgggtagcac aaattagaaa caaacctaat aattatgaaa cgtattattc agattctttg    1140
   aaaggcagat tcaccatctc aagagatgat tccaaaagta gtgtctacct gcaaatgaac    1200
   aacttaagac ctgaagacat gggtatctat tactgtacac taggtaactc ctggtttgct    1260
   tactggggcc aagggactct ggtcactgtc tctggtggtg gtggatccga cattgtgctg    1320
   acccagtctc cactctccct gcctgtcagt cttggagatc aagcctccat ctcttgcaga    1380
   tctagtcaga gccttgtaca cagtaatgga aacacctatt tacattggta cctgcagaag    1440
   ccaggccagt ctccaaagct cctgatctac aaagtttcca accgattttc tggggtccca    1500
   gacaggttca gtggcagtgg atcagggaca gatttcacac tcaagatcag cagagtggag    1560
   gctgaggatc tgggagttta tttctgctct caaagtacac atgttccatt cacgttcggc    1620
   tcggggacaa agttggaaat aaaacgggct gatgctgcac caactgtatc cgcggccgct    1680
   cgaggagggc ccgaacaaaa actcatctca gaagaggatc tgaatagcgc cgtcgaccat    1740
   catcatcatc atcattga                                                  1758
<210> 33
   <211> 585
   <212> PRT
   <213> Artificial Sequence
<220>
   <223> Fusionsprotein
<400> 33
   Met Glu Thr Asp Thr Leu Leu Leu Trp Val Leu Leu Leu Trp Val Pro
   1               5                   10                  15
   Gly Ser Thr Gly Asp Ala Ala Gln Pro Ala Met Ala Asp Tyr Lys Gln
               20                  25                  30
   Val Lys Leu Gln Gln Ser Gly Ala Glu Leu Val Lys Pro Gly Ala Ser
           35                  40                  45
   Val Lys Leu Ser Cys Lys Ala Ser Gly Tyr Thr Phe Thr Thr Tyr Tyr
       50                  55                  60
   Met Tyr Trp Val Lys Gln Arg Pro Gly Gln Gly Leu Glu Trp Ile Gly
   65                  70                  75                  80
   Glu Ile Ser Pro Ile Asn Gly Ala Thr Asn Phe Asn Glu Lys Phe Lys
                   85                  90                  95
   Ser Lys Ala Thr Leu Thr Val Asp Lys Ser Ser Ser Thr Ala Tyr Met
               100                 105                 110
   Gln Leu Ser Ser Leu Thr Ser Glu Asp Ser Ala Val Tyr Tyr Cys Thr
           115                 120                 125
   Thr Leu Gly Glu Lys Gly Phe Ala Tyr Trp Gly Gln Gly Thr Leu Val
       130                 135                 140
   Thr Val Ser Ala Glu Ser Gln Ser Phe Pro Asn Val Gly Gly Gly Gly
   145                 150                 155                 160
   Ser Asp Ile Val Leu Thr Gln Ser Pro Ser Ser Leu Ala Val Ser Ala
                   165                 170                 175
   Gly Glu Lys Val Thr Met Ser Cys Arg Ser Ser Gln Ser Leu Leu Asp
               180                 185                 190
   Ser Arg Thr Arg Lys Asn Tyr Leu Ala Trp Tyr Gln Gln Lys Pro Gly
           195                 200                 205
   Gln Ser Pro Lys Leu Leu Ile Tyr Trp Ala Ser Thr Arg Glu Ser Gly
       210                 215                 220
   Val Pro Asp Arg Phe Thr Gly Ser Gly Ser Gly Thr Asp Phe Thr Leu
   225                 230                 235                 240
   Thr Ile Ser Asn Val Gln Ala Glu Asp Leu Ala Val Tyr Tyr Cys Lys
                   245                 250                 255
   Gln Ser Tyr Asn Leu Pro Thr Phe Gly Gly Gly Thr Lys Leu Glu Ile
               260                 265                 270
   Lys Arg Ala Asp Ala Ala Pro Thr Val Ser Gly Pro Gly Gly Gly Gly
           275                 280                 285
   Ser Gly Gly Gly Gly Ser Gly Ala Gly Gly Ser Gly Gly Gly Gly Ser
       290                 295                 300
   Gly Gly Gly Gly Ser Met Ala Asp Tyr Lys Glu Val Lys Leu Gln Glu
   305                 310                 315                 320
   Ser Gly Gly Gly Leu Val Gln Pro Gly Arg Pro Met Lys Leu Ser Cys
               325                 330                 335
   Val Ala Ser Gly Phe Thr Phe Ser Asp Phe Trp Met Asn Trp Val Arg
           340                 345                 350
   Gln Ser Pro Glu Lys Gly Leu Glu Trp Val Ala Gln Ile Arg Asn Lys
       355                 360                 365
   Pro Asn Asn Tyr Glu Thr Tyr Tyr Ser Asp Ser Leu Lys Gly Arg Phe
   370                 375                 380
   Thr Ile Ser Arg Asp Asp Ser Lys Ser Ser Val Tyr Leu Gln Met Asn
   385                 390                 395                 400
   Asn Leu Arg Pro Glu Asp Met Gly Ile Tyr Tyr Cys Thr Leu Gly Asn
                   405                 410                 415
   Ser Trp Phe Ala Tyr Trp Gly Gln Gly Thr Leu Val Thr Val Ser Gly
               420                 425                 430
   Gly Gly Gly Ser Asp Ile Val Leu Thr Gln Ser Pro Leu Ser Leu Pro
           435                 440                 445
   Val Ser Leu Gly Asp Gln Ala Ser Ile Ser Cys Arg Ser Ser Gln Ser
       450                 455                 460
   Leu Val His Ser Asn Gly Asn Thr Tyr Leu His Trp Tyr Leu Gln Lys
   465                 470                 475                 480
   Pro Gly Gln Ser Pro Lys Leu Leu Ile Tyr Lys Val Ser Asn Arg Phe
                   485                 490                 495
   Ser Gly Val Pro Asp Arg Phe Ser Gly Ser Gly Ser Gly Thr Asp Phe
               500                 505                 510
   Thr Leu Lys Ile Ser Arg Val Glu Ala Glu Asp Leu Gly Val Tyr Phe
           515                 520                 525
   Cys Ser Gln Ser Thr His Val Pro Phe Thr Phe Gly Ser Gly Thr Lys
       530                 535                 540
   Leu Glu Ile Lys Arg Ala Asp Ala Ala Pro Thr Val Ser Ala Ala Ala
   545                 550                 555                 560
   Arg Gly Gly Pro Glu Gln Lys Leu Ile Ser Glu Glu Asp Leu Asn Ser
                   565                 570                 575
   Ala Val Asp His His His His His His
               580                 585
<210> 34
   <211> 1113
   <212> DNA
   <213> Artificial Sequence
<220>
   <223> Fusionsprotein
<400> 34
   atgggcagca gccatcatca tcatcatcac agcagcggcc tggtgccgcg cggcagccat      60
   atggctagcg agaaagaagc actgaagaaa ataatagaag accaacaaga atccctaaac     120
   aaaactagca tgactggtgg acagcaaatg ggtcgggatc cgagacgaaa ttattctctt     180
   gataaaatta ttgttgatta taatcttcaa tctaaaatta ctcttcctcg taggaagctt     240
   gcggccgcac tcgagaaaga agcactgaag aaaataatag aagaccaaca agaatcccta     300
   aacaaaacta gggacgtcat ggtgagcaag ggcgaggagc tgttcaccgg ggtggtgccc     360
   atcctggtcg agctggacgg cgacgtaaac ggccacaagt tcagcgtgtc cggcgagggc     420
   gagggcgatg ccacctacgg caagctgacc ctgaagttca tctgcaccac cggcaagctg     480
   cccgtgccct ggcccaccct cgtgaccacc ctgacctacg gcgtgcagtg cttcagccgc     540
   taccccgacc acatgaagca gcacgacttc ttcaagtccg ccatgcccga aggctacgtc     600
   caggagcgca ccatcttctt caaggacgac ggcaactaca agacccgcgc cgaggtgaag     660
   ttcgagggcg acaccctggt gaaccgcatc gagctgaagg gcatcgactt caaggaggac     720
   ggcaacatcc tggggcacaa gctggagtac aactacaaca gccacaacgt ctatatcatg     780
   gccgacaagc agaagaacgg catcaaggtg aacttcaaga tccgccacaa catcgaggac     840
   ggcagcgtgc agctcgccga ccactaccag cagaacaccc ccatcggcga cggccccgtg     900
   ctgctgcccg acaaccacta cctgagcacc cagtccgccc tgagcaaaga ccccaacgag     960
   aagcgcgatc acatggtcct gctggagttc gtgaccgccg ccgggatcac tctcggcatg    1020
   gacgtcagta ctgagaaaga agcactgaag aaaataatag aagaccaaca agaatcccta    1080
   aacaaaacta gtcaccacca ccaccaccac tga                                 1113
<210> 35
   <211> 370
   <212> PRT
   <213> Artificial Sequence
<220>
   <223> Fusionsprotein
<400> 35
   Met Gly Ser Ser His His His His His His Ser Ser Gly Leu Val Pro
   1               5                   10                  15
   Arg Gly Ser His Met Ala Ser Glu Lys Glu Ala Leu Lys Lys Ile Ile
               20                  25                  30
   Glu Asp Gln Gln Glu Ser Leu Asn Lys Thr Ser Met Thr Gly Gly Gln
           35                  40                  45
   Gln Met Gly Arg Asp Pro Arg Arg Asn Tyr Ser Leu Asp Lys Ile Ile
       50                  55                  60
   Val Asp Tyr Asn Leu Gln Ser Lys Ile Thr Leu Pro Arg Arg Lys Leu
   65                  70                  75                  80
   Ala Ala Ala Leu Glu Lys Glu Ala Leu Lys Lys Ile Ile Glu Asp Gln
                   85                  90                  95
   Gln Glu Ser Leu Asn Lys Thr Arg Asp Val Met Val Ser Lys Gly Glu
               100                 105                 110
   Glu Leu Phe Thr Gly Val Val Pro Ile Leu Val Glu Leu Asp Gly Asp
           115                 120                 125
   Val Asn Gly His Lys Phe Ser Val Ser Gly Glu Gly Glu Gly Asp Ala
       130                 135                 140
   Thr Tyr Gly Lys Leu Thr Leu Lys Phe Ile Cys Thr Thr Gly Lys Leu
   145                 150                 155                 160
   Pro Val Pro Trp Pro Thr Leu Val Thr Thr Leu Thr Tyr Gly Val Gln
                   165                 170                 175
   Cys Phe Ser Arg Tyr Pro Asp His Met Lys Gln His Asp Phe Phe Lys
               180                 185                 190
   Ser Ala Met Pro Glu Gly Tyr Val Gln Glu Arg Thr Ile Phe Phe Lys
           195                 200                 205
   Asp Asp Gly Asn Tyr Lys Thr Arg Ala Glu Val Lys Phe Glu Gly Asp
       210                 215                 220
   Thr Leu Val Asn Arg Ile Glu Leu Lys Gly Ile Asp Phe Lys Glu Asp
   225                 230                 235                 240
   Gly Asn Ile Leu Gly His Lys Leu Glu Tyr Asn Tyr Asn Ser His Asn
                   245                 250                 255
   Val Tyr Ile Met Ala Asp Lys Gln Lys Asn Gly Ile Lys Val Asn Phe
               260                 265                 270
   Lys Ile Arg His Asn Ile Glu Asp Gly Ser Val Gln Leu Ala Asp His
           275                 280                 285
   Tyr Gln Gln Asn Thr Pro Ile Gly Asp Gly Pro Val Leu Leu Pro Asp
   290 295 300
   Asn His Tyr Leu Ser Thr Gln Ser Ala Leu Ser Lys Asp Pro Asn Glu
   305 310 315 320
   Lys Arg Asp His Met Val Leu Leu Glu Phe Val Thr Ala Ala Gly Ile
   325 330 335
   Thr Leu Gly Met Asp Val Ser Thr Glu Lys Glu Ala Leu Lys Lys Ile
   340 345 350
   Ile Glu Asp Gln Gln Glu Ser Leu Asn Lys Thr Ser His His His His
   355 360 365
   His His
   370
<210> 36
   <211> 1059
   <212> DNA
   <213> Artificial Sequence
<220>
   <223> Fusionsprotein
<400> 36
   atgggcagca gccatcatca tcatcatcac agcagcggcc tggtgccgcg cggcagccat      60
   atggctagcg agaaagaagc actgaagaaa ataatagaag accaacaaga atccctaaac     120
   aaaactagca tgactggtgg acagcaaatg ggtcgggatc cgaattcgag ctccgtcgac     180
   aagcttgcgg ccgcactcga gaaagaagca ctgaagaaaa taatagaaga ccaacaagaa     240
   tccctaaaca aaactaggga cgtcatggtg agcaagggcg aggagctgtt caccggggtg     300
   gtgcccatcc tggtcgagct ggacggcgac gtaaacggcc acaagttcag cgtgtccggc     360
   gagggcgagg gcgatgccac ctacggcaag ctgaccctga agttcatctg caccaccggc     420
   aagctgcccg tgccctggcc caccctcgtg accaccctga cctacggcgt gcagtgcttc     480
   agccgctacc ccgaccacat gaagcagcac gacttcttca agtccgccat gcccgaaggc     540
   tacgtccagg agcgcaccat cttcttcaag gacgacggca actacaagac ccgcgccgag     600
   gtgaagttcg agggcgacac cctggtgaac cgcatcgagc tgaagggcat cgacttcaag     660
   gaggacggca acatcctggg gcacaagctg gagtacaact acaacagcca caacgtctat     720
   atcatggccg acaagcagaa gaacggcatc aaggtgaact tcaagatccg ccacaacatc     780
   gaggacggca gcgtgcagct cgccgaccac taccagcaga acacccccat cggcgacggc     840
   cccgtgctgc tgcccgacaa ccactacctg agcacccagt ccgccctgag caaagacccc     900
   aacgagaagc gcgatcacat ggtcctgctg gagttcgtga ccgccgccgg gatcactctc     960
   ggcatggacg tcagtactga gaaagaagca ctgaagaaaa taatagaaga ccaacaagaa    1020
   tccctaaaca aaactagtca ccaccaccac caccactga                           1059
<210> 37
   <211> 352
   <212> PRT
   <213> Artificial Sequence
<220>
   <223> Fusionsprotein
<400> 37
   Met Gly Ser Ser His His His His His His Ser Ser Gly Leu Val Pro
   1               5                   10                  15
   Arg Gly Ser His Met Ala Ser Glu Lys Glu Ala Leu Lys Lys Ile Ile
               20                  25                  30
   Glu Asp Gln Gln Glu Ser Leu Asn Lys Thr Ser Met Thr Gly Gly Gln
           35                  40                  45
   Gln Met Gly Arg Asp Pro Asn Ser Ser Ser Val Asp Lys Leu Ala Ala
       50                  55                  60
   Ala Leu Glu Lys Glu Ala Leu Lys Lys Ile Ile Glu Asp Gln Gln Glu
   65                  70                  75                  80
   Ser Leu Asn Lys Thr Arg Asp Val Met Val Ser Lys Gly Glu Glu Leu
                   85                  90                  95
   Phe Thr Gly Val Val Pro Ile Leu Val Glu Leu Asp Gly Asp Val Asn
               100                 105                 110
   Gly His Lys Phe Ser Val Ser Gly Glu Gly Glu Gly Asp Ala Thr Tyr
           115                 120                 125
   Gly Lys Leu Thr Leu Lys Phe Ile Cys Thr Thr Gly Lys Leu Pro Val
       130                 135                 140
   Pro Trp Pro Thr Leu Val Thr Thr Leu Thr Tyr Gly Val Gln Cys Phe
   145                 150                 155                 160
   Ser Arg Tyr Pro Asp His Met Lys Gln His Asp Phe Phe Lys Ser Ala
                   165                 170                 175
   Met Pro Glu Gly Tyr Val Gln Glu Arg Thr Ile Phe Phe Lys Asp Asp
               180                 185                 190
   Gly Asn Tyr Lys Thr Arg Ala Glu Val Lys Phe Glu Gly Asp Thr Leu
           195                 200                 205
   Val Asn Arg Ile Glu Leu Lys Gly Ile Asp Phe Lys Glu Asp Gly Asn
       210                 215                 220
   Ile Leu Gly His Lys Leu Glu Tyr Asn Tyr Asn Ser His Asn Val Tyr
   225                 230                 235                 240
   Ile Met Ala Asp Lys Gln Lys Asn Gly Ile Lys Val Asn Phe Lys Ile
                   245                 250                 255
   Arg His Asn Ile Glu Asp Gly Ser Val Gln Leu Ala Asp His Tyr Gln
               260                 265                 270
   Gln Asn Thr Pro Ile Gly Asp Gly Pro Val Leu Leu Pro Asp Asn His
           275                 280                 285
   Tyr Leu Ser Thr Gln Ser Ala Leu Ser Lys Asp Pro Asn Glu Lys Arg
       290                 295                 300
   Asp His Met Val Leu Leu Glu Phe Val Thr Ala Ala Gly Ile Thr Leu
   305                 310                 315                 320
   Gly Met Asp Val Ser Thr Glu Lys Glu Ala Leu Lys Lys Ile Ile Glu
                   325                 330                 335
   Asp Gln Gln Glu Ser Leu Asn Lys Thr Ser His His His His His His
               340                 345                 350
<210> 38
   <211> 34
   <212> DNA
   <213> Artificial Sequence
<220>
   <223> Primer
<220>
   <221> misc_feature
   <222> (17)..(17)
   <223> n is a, c, g, or t
<400> 38
   tttttggatc csargtnmag ctgsagsagt cwgg 34
<210> 39
   <211> 30
   <212> DNA
   <213> Artificial Sequence
<220>
   <223> Primer
<400> 39
   attgggacta gtttctgcga cagctggatt 30
<210> 40
   <211> 32
   <212> DNA
   <213> Artificial Sequence
<220>
   <223> Primer
<400> 40
   ggaagatctc ttgaccaggc atcctagagt ca 32
<210> 41
   <211> 36
   <212> DNA
   <213> Artificial Sequence
<220>
   <223> Primer
<400> 41
   tttttgaatt ctgayattgt gmtsacmcar wctmca 36
<210> 42
   <211> 32
   <212> DNA
   <213> Artificial Sequence
<220>
   <223> Primer
<400> 42
   tttttgggcc cggatacagt tggtgcagca tc 32
<210> 43
   <211> 40
   <212> DNA
   <213> Artificial Sequence
<220>
   <223> Primer
<400> 43
   ccatggcgga ctacaaagat attgtgctga cccagtctcc 40
<210> 44
   <211> 53
   <212> DNA
   <213> Artificial Sequence
<220>
   <223> Primer
<400> 44
   ccagaaccac caccaccaga accaccacca ccggatacag ttggtgcagc atc 53
<210> 45
   <211> 58
   <212> DNA
   <213> Artificial Sequence
<220>
   <223> Primer
<400> 45
   tggtggttct ggcggcggcg gctccggtgg tggtggatcc gaagttaagc tggaggag 58
<210> 46
   <211> 28
   <212> DNA
   <213> Artificial Sequence
<220>
   <223> Primer
<400> 46
   gcggccgcga catttgggaa ggactgac 28
<210> 47
   <211> 52
   <212> DNA
   <213> Artificial Sequence
<220>
   <223> Primer
<400> 47
   cggcccagcc ggccatggcg gactacaaag aagttaagct gcaggagtca gg 52
<210> 48
   <211> 50
   <212> DNA
   <213> Artificial Sequence
<220>
   <223> Primer
<400> 48
   gagccgccgc cgccagaacc accaccacca gagacagtga ccagagtccc 50
<210> 49
   <211> 53
   <212> DNA
   <213> Artificial Sequence
<220>
   <223> Primer
<400> 49
   ggcggcggcg gctccggtgg tggtggatcc gacattgtga tcacacagtc tcc 53
<210> 50
   <211> 29
   <212> DNA
   <213> Artificial Sequence
<220>
   <223> Primer
<400> 50
   gcggccgcgg atacagttgg tgcagcatc 29
<210> 51
   <211> 66
   <212> DNA
   <213> Artificial Sequence
<220>
   <223> Primer
<400> 51
   ggcgccggcg gctccggtgg tggtggttcc ggtggtggtg gctccatggc ggactacaaa 60
   gaggtc 66
<210> 52
   <211> 41
   <212> DNA
   <213> Artificial Sequence
<220>
   <223> Primer
<400> 52
   ggatccacca ccaccagaga cagtgaccag agtcccttgg c 41
<210> 53
   <211> 28
   <212> DNA
   <213> Artificial Sequence
<220>
   <223> Primer
<400> 53
   ggatccgaca ttgtgctcac acagtctc 28
<210> 54
   <211> 66
   <212> DNA
   <213> Artificial Sequence
<220>
   <223> Primer
<400> 54
   ggcgccagaa ccaccaccac cagaaccacc accaccgggc ccggatacag ttggtgcagc        60
   atcagc                                                                   66
<210> 55
   <211> 20
   <212> PRT
   <213> Clostridium tetani
<400> 55
   Asn Tyr Ser Leu Asp Lys Ile Ile Val Asp Tyr Asn Leu Gln Ser Lys
   1               5                   10                  15
   Ile Thr Leu Pro
               20